# EUROPEAN PATENT APPLICATION

(11) **EP 1 760 079 A1**
(43) Date of publication of application: **07.03.2007**
(21) Application number: 05745210.4
(22) Date of filing: 12.05.2005
(51) Int. Cl.: C07D 401/06, C07D 403/08, C07D 413/08, A61K 31/4025, A61K 31/435, A61P 31/18, A61P 1/00, A61P 17/00, A61P 19/00

(54) **COMPOUNDS AS COR5 ANTAGONISTS**

(30) Priority: 09.06.2004 CN 200410025006
(71) Applicant: Shanghai Target Drug Co., Ltd., Shanghai 200233 (CN)
(72) Inventor: PEI, Gang, Shanghai 200233 (CN); MA, Dawei, Shanghai 200233 (CN); CHEN, Li, Shanghai 200233 (CN); YU, Shanghai, Shanghai 200233 (CN); LI, Ben, Shanghai 200233 (CN); DONG, Fang, Shanghai 200233 (CN); LV, Yanhui, Shanghai 200233 (CN); CHEN, Renhai, Shanghai 200233 (CN); ZHANG, Jin, Shanghai 200233 (CN)
(74) Representative: Esteban Perez-Serrano, Maria Isabel
(86) International application number: PCT/CN2005/000659
(87) International publication number: WO 2005/121123

(57) **Abstract**

The present invention discloses the compounds of formula I or their pharmaceutically acceptable salts, which are useful as CCR5 antagonists. The preparation and use of the compounds of formula I, pharmaceutical composition containing the same are also disclosed. Furthermore, the present invention discloses an intermediate for the preparation of the compounds of formula I.

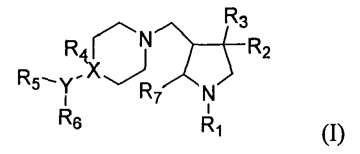

## Description

### Field of the Invention

This invention relates to compounds (pyrrolidine derivatives) useful as CCR5 receptor antagonists, the preparation methods and the uses thereof.

### Background of the Invention

Chemokines are a family of cytokines that mediate directional migration of lymphocytes. They play a major role in inflammatory responses, leucocyte exosmosis, tissue infiltration, tumorigenesis and embryonic development. Chemokines belong to a family of secreted signal molecules, with molecular weights ranging from 8 kD to 14 kD. Currently there are about 45 members in the family, which share the common characteristic, i.e. contain four position-conserved cysteines. According to the presence of intervening amino acid(s) between the two conserved cysteines near the N terminal, the chemokine family is grouped into 4 categories: C-X-C, C-C, C-X₃-C and C chemokines, among which C-X-C chemokines (also α-chemokines) and the C-C chemokines (also (β-chemokines) are the major members.

The function of chemokines is mediated by chemokine receptors, which are named according to the characteristics of specifically binding chemokines (for example, if its ligand is C-C chemokine sub-family, then the receptor is named CCR). Chemokine receptors are G protein coupled receptors (GPCR), which have seven conserved alpha helix transmembrane domains as well as an extracellular N terminal and an intracellular C terminal. Upon binding to agonists, this receptor family can couple to G protein, and mediate the signal transduction. With the effect of the agonists, chemokine receptors can induce a series of intracellular signals and change the behavior of cells, such as inhibition of adenosine cyclase, mobilize intracellular calcium release, activate a series of protein kinases, induce cell directional migration (chemotaxis) and affect the secretion of cytokines.

So far, 19 chemokine receptors have been identified: CCR1-11, CXCR1-6, XCR1 and CX₃CR1. Chemokine receptors are regarded as important mediators of inflammatory responses and autoimmune diseases (Gerard et al, Nat Immunol, 2,108-15 (2001)), therefore, the modulators of chemokine receptors (including agonists and antagonists) can be used in various diseases such as inflammation, allergy, autoimmune diseases, inflammatory intestine diseases, scleroderma, eosinophilic myositis, tumorigenesis and metastasis *etc.*

CCR5 is one of the chemokine receptors, and its endogenous agonists are RANTES, MIP-1α and MIP-1β. CCR5 expresses on dendritic cells from the peripheral blood, T lymphocytes, mononuclear cells, macrophages and immune cells and inflammatory cells that participate in maintenance of long-term inflammation. Therefore, the modulation of CCR5 function could regulate the recruitment of T cells to inflammatory sites, making CCR5 a new treatment target for inflammation and autoimmune diseases. For example, CCR5 deficiency protected mice from DSS induced severe inflammation and mucosa injury (Andres et al., J Immunol, 164, 6303-12, (2002)); TAK779, a small molecule antagonist of CCR5 inhibited collagen-induced arthritis in mice (Yang et al., Eur. J Immunol, 32,2124-32, (2002)). Therefore, CCR5 antagonists can be used to treat asthma, local disorder (such as local dermatitis, local anaphylaxis), rheumatoid arthritis, atherosclerosis, psoriasis, sarcoid, other fibrosis diseases and autoimmune diseases (such as multiple sclerosis, inflammatory enteronitis). Also, CD⁸⁺T cell is related to chronic obstructive pulmonary diseases (COPD) (Cosio et al., Chest, 121, 160S-165S, (2002)), therefore, CCR5 antagonists may be applied to COPD treatment.

Besides its roles in inflammatory and immune responses, chemokine receptors may also be important for some virus and parasites to enter the cells. For example, Duffy receptor mediates plasmodia entry to red blood cells, and individuals deficient in the Duffy receptor are protected from malaria. More importantly, some chemokine receptors take part in HIV invasion, and are therefore called HIV co-receptors.

Studies showed that the CD4 molecule on the Th cells is indispensable for HIV invasion, but CD4 alone is not sufficient to mediate the confluence of HIV with cells. Further studies revealed that, the other so-called HIV invasion co-receptors are chemokine receptors CCR5, CXCR4, CCR2b, CCR3, CCR8 and orphan receptor V28, STRL33, GPR1, GPR15 and APJ (Doms et al., Virology, 235, 179-90, (1997)). CCR5 and CXCR4 are the major HIV co-receptors in vivo for HIV invasion, while CCR3 may partially take part in the HIV entry process. CCR5 is macrophage tropic (M-tropic) HIV-1 co-receptor while CXCR4 is the T cell tropic (T-tropic) HIV-1 co-receptor. Therefore, CCR5 is crucial for HIV transmission, and CCR5 modulators can regulate the transmission of M tropic HIV-1 in human beings and control the disease at an early stage. *In vitro* data also proved that, CCR5 binding chemokines-RANTES, MIP-1α and MIP-1β can block the entry of HIV-1 into cells and thus inhibit the HIV infection. Small molecule drugs that can bind to CCR5 and antagonize its function can also effectively inhibit HIV entry in vitro.

As described above, there is an urgent need to develop a new class of compounds useful as potent CCR5 antagonists.

### DISCLOSURE OF THE INVENTION

One object of the invention is to provide a class of compounds useful as CCR5 antagonists.

Another object of the invention is to provide the production processes for the compounds and the uses of the compounds.

In the first aspect, the invention provides compound of formula I or pharmaceutically acceptable salts thereof; 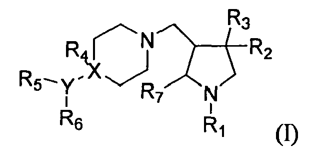 wherein R₁ is benzyl, benzoyl, cyclohexanecarbonyl, cyclopentanecarbonyl, phenylsulfonyl or naphthylcarbonyl, the groups are optionally substituted with 1-3 substituents independently selected from the group consisting of halogen, C₁₋₄ alkyl and C₁₋₄ alkoxy;
wherein R₂ is hydroxyl, phenylcarbonyloxy, phenoxyl, thiophenyl, anilino or phenylsulfonyl, wherein the benzene rings of the groups are optionally substituted with 1-3 substituents independently selected from the group consisting of halogen and C₁₋₄ alkyl;
wherein R₃ is hydrogen, C₁₋₄ alkyl, phenyl or 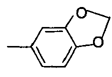 (benzo[d][1,3]dioxol-5-yl), wherein the benzene rings of the groups are optionally substituted with 1-3 substituents independently selected from the group consisting of halogen and C₁₋₄ alkyl;
wherein R₄ is hydrogen, hydroxyl or is absent;
wherein R₇ is hydrogen, C₁₋₆ alkyl or phenyl;
wherein X is oxygen or carbon or is absent;
provided that when X is oxygen or is absent, R₄, R₅, R₆ or Y are absent; or
provided when X is carbon, Y is nitrogen, R₅ is C₁₋₆ alkyl or allyl and R₆ is selected from the group consisting of 4-nitro benzyloxycarbonyl, benzyloxycarbonyl, 4-halogen benzyloxycarbonyl, 4-methoxy benzyloxycarbonyl, 4-methyl benzyloxycarbonyl, 4-trifluoromethyl benzyloxycarbonyl, 4-amino benzyloxycarbonyl; benzo[d][1,3]dioxol-5-yl methyloxycarbonyl, phenylsulfonyl, 4-methyl phenylsulfonyl, 2-phenoxyacetyl and phenylcarbamyl. Or R₅, R₆ and Y together form phenyl or -R₈-phenyl, wherein R₈ is C₁₋₄ alkylidene;

Preferred are compounds of formula I wherein R₁ is benzyl, benzoyl, m-halogen benzoyl, cyclohexanecarbonyl, cyclopentanecarbonyl, phenylsulfonyl or naphthylcarbonyl.

Also preferred are compounds of formula I wherein R₂ is hydroxyl, phenylcarbonyloxy, phenoxyl, thiophenyl, anilin or phenylsulfonyl.

Also preferred are compounds of formula I wherein R₃ is hydrogen, C₁₋₄ alkyl, phenyl, 4-halogen phenyl, or 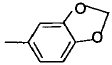 (benzo[d][1,3]dioxol-5-yl).

Also preferred are compounds of formula I, wherein X is oxygen or is absent and R₄, R₅, R₆ and Y are absent.

Also preferred are compounds of formula I wherein X is carbon, Y is nitrogen, R₅ is C₁₋₆ alkyl or allyl and R₆ is selected from the group consisting of 4-nitro benzyloxycarbonyl, benzyloxycarbonyl, 4-halogen benzyloxycarbonyl, 4-methoxyl benzyloxycarbonyl, 4-methyl benzyloxycarbonyl, 4-trifluoromethyl benzyloxycarbonyl, 4-amino benzyloxycarbonyl; benzo[d][1,3]dioxol-5-yl methyloxycarbonyl, phenylsulfonyl, 4-methyl phenylsulfonyl, 2-phenoxyacetyl and phenylcarbamyl; Or R₅, R₆ and Y together form phenyl or -CH₂CH₂CH₂-phenyl .

Also preferred are compounds of formula I wherein R₇ is hydrogen, C₁₋₃ alkyl or phenyl.

Also preferred are compounds of formula III, 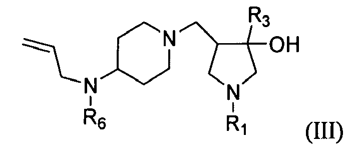 wherein,
R₁ is benzyl, benzoyl, m-halogen benzoyl, cyclohexanecarbonyl, cyclopentanecarbonyl, phenylsulfonyl or naphthylcarbonyl, the groups are optionally substituted with 1-3 substituents independently selected from the group consisting of halogen, C₁₋₄ alkyl and C₁₋₄ alkoxy;
R₃ is hydrogen, C₁₋₄ alkyl, phenyl or 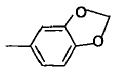 (benzo[d][1,3]dioxol-5-yl), wherein the benzene groups of the groups are optionally substituted with 1, 2 or 3 substituents independently selected from the group consisting of halogen and C₁₋₄ alkyl;
R₆ is selected from the group consisting of 4-nitro benzyl oxycarbonyl, benzyloxycarbonyl, 4-halogen benzyloxycarbonyl, 4-methoxyl benzyloxycarbonyl, 4-methyl benzyloxycarbonyl, 4-trifluoromethyl benzyloxycarbonyl, 4-amino benzyloxycarbonyl; benzo[d][1,3]dioxol-5-yl methyloxycarbonyl, phenylsulfonyl, 4-methyl phenylsulfonyl, 2-phenoxyacetyl and phenylcarbamyl.

Most preferred compounds are listed in Table I.

In another aspect, the invention provides a pharmaceutical composition comprising compound of formula I in combination with a pharmaceutically acceptable carrier.

In another aspect, the invention provides the use of compound of formula I in the preparation of a medicament for treating HIV infection, asthma, rheumatoid arthritis, autoimmune diseases and chronic obstructive pulmonary diseases (COPD).

In another aspect, the invention provides an intermediate of formula II useful to prepare compound of formula I, 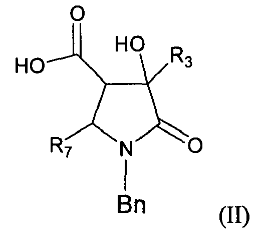 wherein,
R₃ is hydrogen, C₁₋₄ alkyl, phenyl or 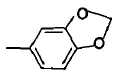 (benzo[d][1,3]dioxol-5-yl), wherein the benzene rings of the groups are optionally substituted with 1-3 substituents independently selected from the group consisting of halogen, C₁₋₄ alkyl;
R₇ is hydrogen, C₁₋₆ alkyl or phenyl.

### DETAILED DESCRIPTION OF THE INVENTION

After intensive and extensive study, the inventors designed and synthesized a class of pyrrolidine derivatives based on CCR5' structural features. The results of all tests demonstrated that these compounds were potent CCR5 antagonists. The inventors completed the present invention based on the above.

As used herein, the term "alkyl" is intended to include both branched and straight-chain saturated aliphatic hydrocarbon groups having 1-8, preferredly 1-6 carbon atoms. "Alkenyl" is intended to include straight or branched hydrocarbon groups having at least one carbon-carbon double bond and 2-8 (preferredly 2-6) carbon atoms. "Alkynyl" is intended to include straight or branched hydrocarbon groups having at least one carbon-carbon triple bond and 2-8 (preferredly 2-6) carbon atoms.

As used herein, the term "aryl" used herein refers to aromatic system and may be monocyclic or polycyclic aryl group fused together or attached together, thus making at least a portion of fused or attached rings forming conjugated aromatic system.

Examples of aryl groups include, but are not limited to, phenyl, naphthyl or tetrohydronaphthyl (tetralin).

As used herein, the term "heterocycle" or "heterocyclic system" is intended to mean a stable 4, 5, 6, 7-membered monocyclic or multicyclic heterocyclic ring which is saturated, partially unsaturated or unsaturated, and which consists of carbon atoms and 1-4 heteroatoms independently selected from the group consisting of N, O and S and including any multicyclic group in which the above-defined heterocyclic rings is fused to an aromatic ring. The nitrogen and sulfur heteroatoms may optionally be oxidized.

As used herein, the term "substituted aryl" or "substituted heterocyclic" refers to an aryl group or a heterocyclic group as defined above having 1 to 4 substituents independently selected from halo, cyano, hydroxy, nitro, amino, alkyl, cycloalkyl, alkenyl, alkynyl, alkoxy, aryloxy, substituted alkoxy, alkylcarbonyl, alkylcarboxy, alkylamino or arylthiol. Preferred substituents are halo and C₁₋₄ alkyl.

"Halo" or "halogen" as used herein refers to fluoro, chloro, bromo or iodo.

The compounds of the present invention may be administered in the form of pharmaceutically or physiologically acceptable salts which are derived from acids or bases. Examples of the salts include, but are not limited to, those derived from inorganic acids such as hydrochloric acid, sulfuric acid, nitric acid, phosphoric acid and the like; and the salts prepared from organic acids such as acetic acid, oxalic acid, succinic acid, tartaric acid, methanesulfonic acid, maleic acid and the like. Examples of the other salt include a salt with alkali metal or alkaline earth metal (e.g. sodium, potassium calcium, magnesium). Examples of the prodrug of the compound of the present invention include ester, carbamate and other conventional forms, which are converted into the active ingredient in vivo when administered in this form. The invention includes a pharmaceutical composition and a method of treatment comprising administering a therapeutically effective amount of compound of formula I to mammals. A compound of the present invention is useful for treating HIV infection, asthma, local disorder (eg: local dermatitis, local anaphylaxis), rheumatoid arthritis, atherosclerosis, psoriasis, sarcoid, other fibrosis diseases and autoimmune diseases (such as multiple sclerosis, inflammatory enteritis) and chronic obstructive pulmonary diseases (COPD). When used for treating the above diseases, the compounds of the present invention may be mixed with one or more pharmaceutically acceptable carriers or excipients, such as solvents and diluents. The compounds of the invention can be administered orally in the form of tablets, capsules, dispersible powders, granules, suspensions (eg. containing about 0.05-5% suspending agent), syrups (eg: containing about 10-50% sugar), elixirs (eg: containing about 20-50% alcohol); or administered parenterally in the form of sterile injectable solutions or suspensions (eg: containing 0.05-5% suspending agent in isotonic medium). For example, these pharmaceutics may contain about 25-90%, generally about 5-60% (by weight) active ingredients, which are mixed with the carriers.

The effective dose level of the active ingredient may vary with the specific compound employed, route of administration and the severity of the disease to be treated. However, when the daily dose of the compounds of this invention is administered in amounts from 0.5 to 500 mg/kg body weight, the effect is generally satisfying. Preferably, 2-4 divided dosages may be administered daily, and the dosage may be administered in slow-released forms. For most large mammals, daily total dosage is about 1-100mg, preferably about 2-80mg. Dosage forms suitable for oral administration include 0.5-500mg active compound mixed with pharmaceutically acceptable solid or liquid carriers. The dosage scheme may be adjusted to provide the best therapeutic response. For example, according to the urgent need to suppress the disease condition, the dosage may be divided to several parts, or the dosage may be reduced proportionally.

These active compounds may be administered orally, intravenously, intramuscularly or subcutaneously. Solid carrier includes: starch, lactose, calcium dihydrogenphosphate, microcrystalline cellulose, sucrose and kaolin, while liquid carrier includes: sterile water, polyethylene glycol, non-ionic surfactant and edible oil (such as corn oil, peanut oil and sesame oil), as long as they are suitable for the active ingredient and the specific administration route. Adjuvants, such as flavoring agent, pigment, preservative and antioxidant, such as vitamin E, vitamin C, BHT and BHA may be advantageously included in the preparation of pharmaceutically composition.

In view of ease to manufacture and administration, the prefeerd pharmaceutically composition is a solid composition, in particular, tablets or capsules filled with solid or liquid. Oral administration of compounds is preferred.

These active compounds may also be administered both parentally and intraperitoneally, and the solution or suspension of the active ingredients (as free base or pharmaceutically acceptable salt) can be manufactured in water mixed with surfactants (such as hydroxypropyl cellulose). Besides, the dispersion may be made in glycerin, liquid, polyethylene glycol and the mixture of polyethylene glycol in oil. Under the condition of regular storage and use, preservatives should be included in the preparations to inhibit the growth of microorganisms.

Dosage forms suitable for injection include: sterile water solution, dispersion and steriled powder (for instant preparation of steriled injectable solution or dispersion). Under all conditions, these dosage forms must be sterile and liquid, for the ejection from the syringes. The dosage forms must be stable under manufacturing and storage conditions, and must be spared the contamination of microorganisms (such as bacteria and fungi). The pharmaceutical carrier can be solvent or dispersing medium, including water, alcohol (such as glycerin, propylene glycol and liquid polyethylene glycol), the appropriate mixtures thereof and vegetable oils.

The compounds of the present invention can be prepared according to the following Schemes.

### Scheme I

Treatement of β-aminopropanoic acid **1** with methanol and SOCl₂ at reflux provides methyl ester. The remaining unprotected amine can then be protected with 2 eq of benzyl bromide in CH₃CN in the presence of K₂CO₃ as a base to give N-protected compound **2.** Aldol condensation of N-protected compound **2** with 2-oxoacetate ester derivative **3** gives two groups of enantiomers **4.**

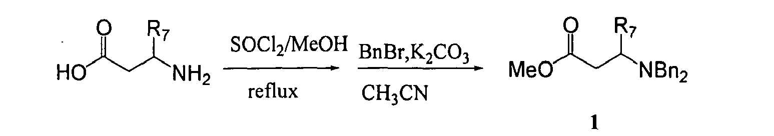

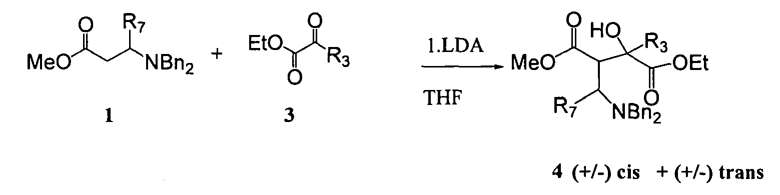

Hydrogenolysis of the condensation product **4** catalyzed by Pd/C in methanol gives rise to two groups of cis/trans isomers of lactam **5** directly. Hydrolysis of methyl ester of **5** with strong base such as NaOH or KOH in methanol affords the same product, its *trans*-acid **6.**

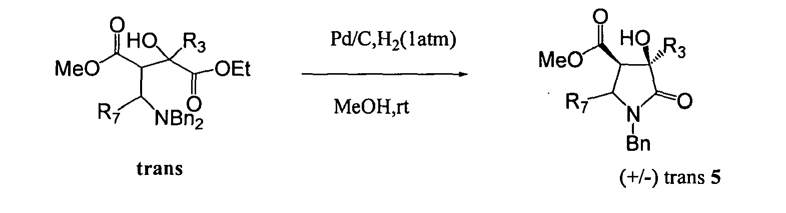

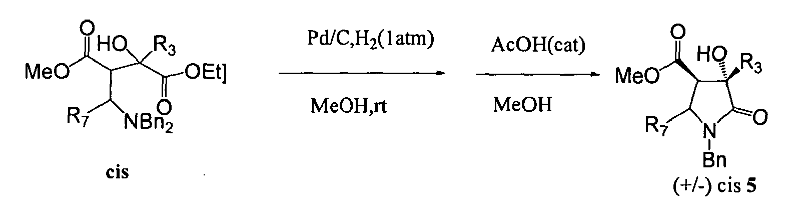

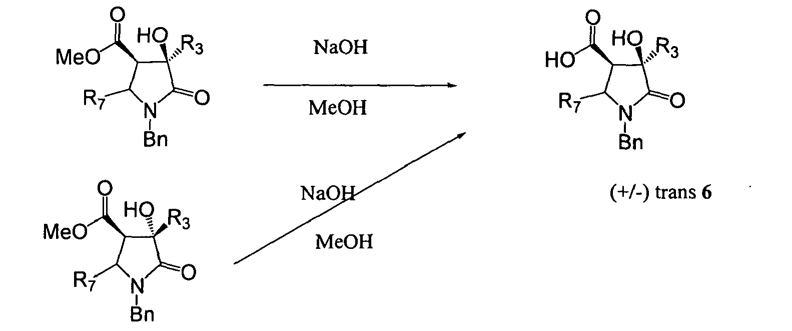

Amide coupling of **6** with amine compounds to afford amide **7** is typically performed in the presence of coupling reagents. The amide **7** is reduced with LAH to provide the desired compound **8**.

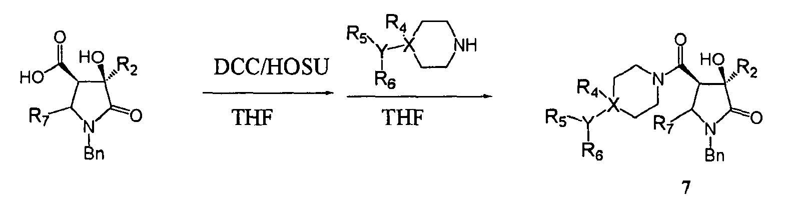

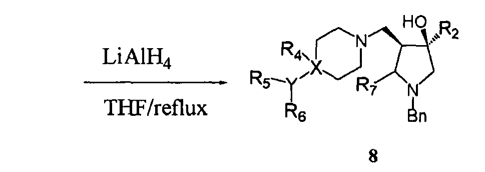

### Scheme II

The benzyl protecting group on compound **8** obtained in Scheme **I** is then removed via Pd/C-catalyzed hydrogenolysis to yield a secondary amine, which is reacted with acyl chlorides to furnish compound **9**.

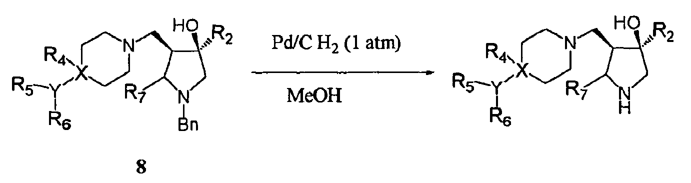

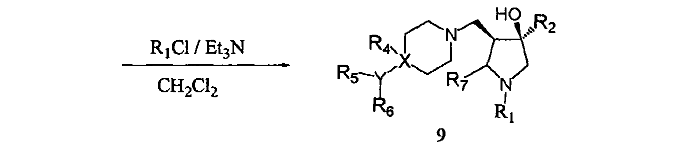

### Scheme III

Amide coupling of acid **6** in Scheme **I** with amine compound **11** to afford amide **12** is typically performed in the presence of coupling reagents. The amide **12** is reduced with LAH to provide the compound **13**, which is reacted with R₆Cl to afford compound **14**.

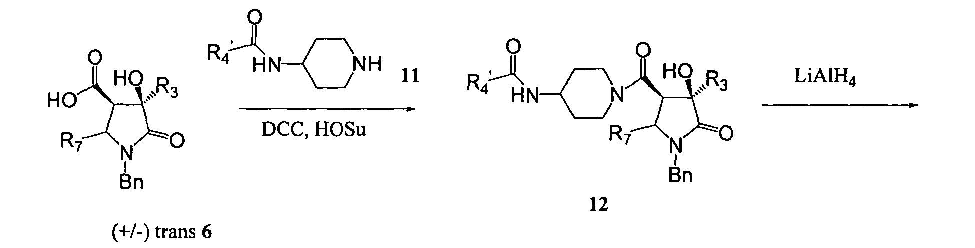

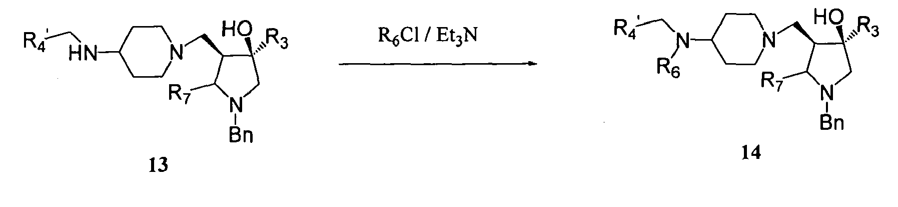

### Scheme IV

Amide coupling of acid **6** in Scheme **I** with piperidine-4,4-diol hydrochloride in the presence of coupling reagents affords ketone **15.** The ketone **15** is reduced with LAH to provide its alcohol derivative **16,** which is Swem oxidized into ketone **17.** Finally, coupling of **17** with amine R₅NH₂ in the presence of NaBH(OAC)₃ results in an intermediate, which is reacted with R₆Cl to furnish the target compound **18.**

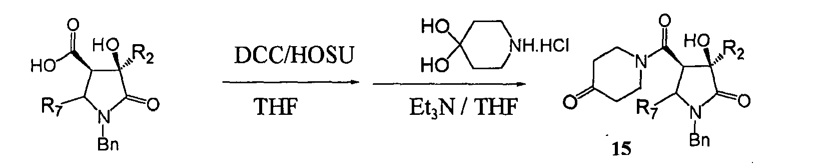

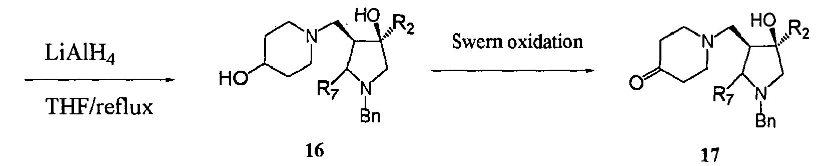

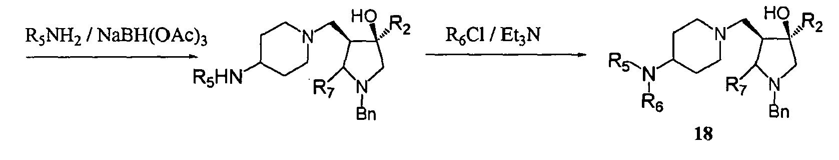

### Scheme V

The secondary alcohol compound **16** in Scheme **IV** is treated with acetic anhydride to afford OH-protected compound **19,** which is hydrogenated to remove the benzyl group to give compound **20.** Compound **20** is reacted with R₁Cl to furnish compound **21.** The acetyl protecting group on compound **21** is then removed via treatment with potassium carbonate in methanol to yield compound **22,** which is Swern oxidized into ketone **23.** Finally, coupling of **23** with amine R₅NH₂ in the presence of NaBH(OAC)₃ results in an intermediate, which is reacted with R₆Cl to furnish the target compound **24.**

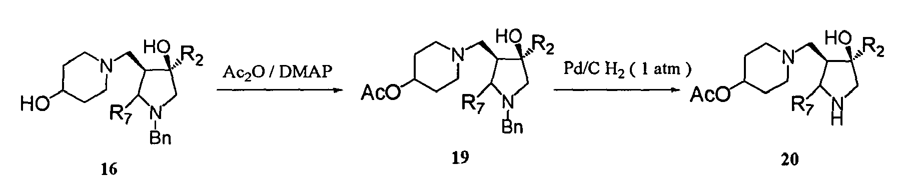

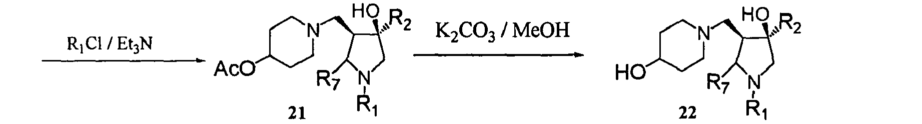

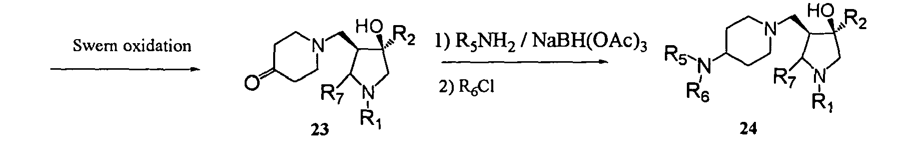

### Scheme VI

Activating the hydroxyl of compound **8** in Scheme **I** with methanesulfonyl chloride affords methanesulfonyl ester, which is reacted with R₂H to give compound **25.**

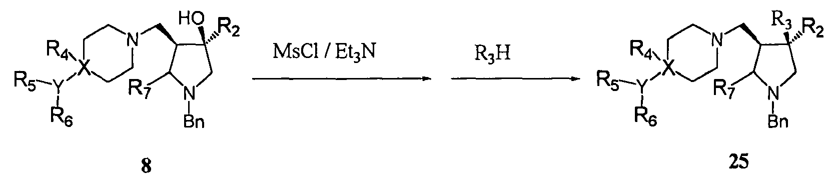

The invention is further illustrated by the following examples. These examples are only intended to illustrate the invention, but not to limit the scope of the invention.

### Example 1

### Compound I-a-a:

### 1-benzyl-3-(benzo[1,3]dioxol-5-yl)-4-[(4-phenylpiperidin-1-yl)methyl]pyrrolidin-3-ol

According to Scheme I, SOCl₂ (10 ml) was added dropwise to the solution of 3-aminopropionic acid hydrochloride (7.05 g) in MeOH (40 ml) in an ice bath. The resulting mixture was refluxed for 3 h, and then cooled and the solvent was evaporated to dryness by rotatary evaporator and then by applying vacuum. K₂CO₃ (38g), CH₃CN (150 ml) were added to the residue with stirring and then BnBr(22 ml) was added. The resulting mixture was stirred for 20 h at room temperature. To the mixture was added water to dissolve K₂CO₃, and the mixture was extracted with ethyl acetate twice. The organic layer was washed with brine, dried over anhydrous sodium sulfate, filtered and concentrated. The residue was chromatographed to give 3-(N,N-dibenzyl)aminopropionic acid methyl ester (14.9 g, 93.6%).
¹H NMR (CDCl₃, 300 MHz) δ 7.27 (m, 10 H), 3.56 (s, 3H), 3.51 (s, 4H), 2.74 (t, *J*= 6.9 Hz, 2H), 2.45 (t, 2H).

### methyl 4-(benzo [1,3]dioxol-5-yl)-1-benzyl-4-hydroxy-pyrrolidin-5-one-3-carboxylate

Butyl lithium (1.6M in hexane, 4.5ml) was added dropwise to the solution of (iPr)₂NH (1.09 ml) in THF (7 ml) at 0 °C under N₂ atmosphere. The mixture was stirred for 10 minutes and cooled to -78 °C and the solution of 3*-(N,N-*dibenzyl)aminopropionic acid methyl ester (1.00g, 3.53mmol) in THF (40 ml) was added dropwise. The reaction mixture was stirred for another 1 h and the solution of ethyl 2-(benzo[1,3]dioxol-5-yl)-2-oxoacetate in THF (5 ml) was added dropwise at -78 °C. Then the mixture was stirred for another 4 h at the same temperature and quenched with saturated NH₄Cl solution and extracted with ethyl acetate (60 ml x 2). The organic layer was washed with brine, dried over sodium sulfate, filtered and concentrated. The residue was chromatographed on silica gel (1/13 EtOAc/hexane) to give a solid compound (0.654 g) and a liquid compound (0.653 g). The total yield was 73.3%.

The liquid compound (3.197 g) was dissolved in methanol (250 ml) and Pd/C (0.32 g) was added to the solution. The mixture was stirred at room temperature for 3 h under 1 atm hydrogen atmosphere. The palladium on carbon was filtered off. The methanol was evaporated to dryness by rotatary evaporator. The residue was chromatographed, eluted with 1:4 EtOAc/hexane to give the unreacted starting compound (1.065 g) and eluted with 1:2 EtOAc/hexane to give a white solid product (0.935 g, 59.8%).
IR (KBr) 3332, 2962, 2916, 1725, 1683, 1504, 1492 cm⁻¹;
¹H NMR (CDC1₃, 300 MHz) δ 7.27-7.41 (m, 5H), 6.76-6.94 (m, 3H), 5.97 (s, 2H), 4.65 (d, *J_{AB}=* 14.7 Hz, 1H), 4.55 (d, *J_{AB} =* 14.7 Hz, 1H), 3.90 (s, 1H), 3.70 (s, 3H), 3.62 (m, 1H), 3.31-3.38 (m, 2H).
ESI-MS *m*/*z* 392 (M⁺+ Na⁺).
Anal. Calcd for C₂₀H₁₉N₁O₆: C, 65.03; H, 5.18; N, 3.79; found: C, 65.11; H, 5.18; N, 3.74.

### 4-(benzo[1,3]dioxol-5-yl)-1-benzyl-4-hydroxy-pyrrolidin-5-one-3-carboxylic acid

1N aqueous sodium hydroxide (1.4 eq) was added to the solution of the above methyl ester compound in methanol. The reaction mixture was reacted at room temperature until the starting compound disappeared. Methanol was removed by rotatory evaporator, water was added, and the solution was acidified to pH 3 with IN aqueous hydrochloric acid. The mixture was extracted with ethyl acetate twice and the organic layer was washed with brine until it was neutral, dried with sodium sulfate, filtered and concentrated to afford a solid compound.
IR (KBr) 3267,2887,1713,1688,1501,1487,1254,1299 cm⁻¹;
EI-MS *m*/*z* (%) 355 (M⁺, 54.49), 337 (2.76), 282 (3.64), 190 (6.62), 149 (100), 119 (19.60), 91 (41.13);
¹H NMR (DMSO *d₆*, 300 MHz) δ 12.45 (s, 1H) 7.41-7.30 (m, 5H), 6.94-6.86 (m, 3H), 6.44 (s, 1H), 6.019 (s, 2H), 4.46 (d, *J_{AB} =* 15.3 Hz, 1H), 4.44 *(d, J_{AB} =* 15.0 Hz, 1 H), 3.5 8 (m, 1H), 3.37 (m, 2H).

### 3-(benzo[1,3]dioxol-5-yl)-1-benzyl-3-hydroxy-4-(4-phenylpiperidine-1-carbonyl)pyrrolidin-2-one

DCC (0.152 g, 0.74 mmol) was added to the solution of the above carboxylic acid compound (0.238 g, 0.67 mmol) and HOSu (0.085 g, 0.74 mmol) in THF (20 ml) at 0 °C under N₂ atmosphere and the resulting mixture was warmed to the room temperature and stirred overnight. The solid was filtered off. 4-phenylpiperidine was added to the filtrate and the reaction mixture was stirred at room temperature for another 12 h. The solvent THF was evaporated by rotatory evaporator and the residue was extracted with ethyl acetate and water and the organic layer was washed with brine, dried, filtered, concentrated and chromatographed (1:1 EtOAc/hexane) to give a white solid (0.152 g, 45%).
IR (KBr) 3325, 2921, 1695, 1682, 1492, 1442 cm⁻¹;
¹H NMR (CDCl₃, 300 MHz) δ 7.31-7.20 (m, 8H), 7.12 (d, 2H), 6.98-6.78 (m, 3H), 5.97 (s, 2H), 4.76 (m, 2H), 4.51 (d, *J_{AB} =* 14.7 Hz, 1H),3.76-3.67 (m, 2H), 3.54 (dd, 1H), 3.38-3.30 (m, 1H), 3.07-2.98 (m, 1H), 2.71-2.60 (m, 2H), 1.88-1.84 (m, 2H), 1.66-1.53 (m, 2H);
EI-MS *m*/*z* (%) 498 (M⁺, 12.79), 480 (67.79), 352(100), 283 (70.60), 189 (95.24), 160 (55.04);
Anal. Calcd for C₃₀H₃₀N₂O₅: C, 72.27; H, 6.06; N, 5.62. found : C, 72.00; H, 5.94; N, 5.56.

### 1-benzyl-3-(benzo[1,3]dioxol-5-yl)-4-[(4-phenylpiperidin-1-yl)methyl]pyrrolidin-3-ol(I-a-a)

LiA1H₄ (8 eq) was added to the solution of the above compound (1 eq) in THF at 0 °C and the mixture was heated under reflux for 24 h. The solution was cooled and quenched with 10% NaOH at 0 °C. The mixture was filtered and THF was evaporated by rotatory evaporator. Ethyl acetate was added to dissolve the residue. The resulting mixture was washed by brine, dried, filtered and concentrated. The residue was chromatographed on silica gel (1:10 Et₃N / EtOAc) to give an oily compound (yield: 90%).
IR (KBr) 2935, 1503,1486 cm⁻¹;
¹H NMR (CDCl₃ 300M Hz) δ 7.32-7.05 (m, 12H), 6.69 (d, *J*= 8.1 Hz, 1H), 5.87 (s, 2H), 3.61 (s, 2H), 2.96-2.88 (m, 3H), 2.82-2.75 (m, 2H), 2.61-2.53 (m, 2H), 2.37-2.32 (m, 4H), 2.08-1.99 (m, 2H), 1.76-1.67 (m,3H);
ESI-MS *m*/*z* 471 (M⁺ + H⁺), 493 (M⁺ + Na⁺).

### Example 2

### Compound I-a-b:

### 1-benzyl-3-(benzo[1,3]dioxol-5-yl)-4-[(4-piperidin-1-yl)methyl]pyrrolidin-3-ol

Compound **I-a-b** was prepared by following the procedure described for the synthesis of compound **I-a-a** via replacement of 4-phenylpiperidine by piperidine.
IR (film) 2933, 1504, 1486 cm⁻¹;
¹H NMR (CDCl₃, 300 MHz) δ 7.38-7.16 (m, 5H), 7.12 (d, *J =* 1.8 Hz, 1H), 7.04 (dd, *J =* 8.1 Hz 1.8 Hz, 1H), 6.68 (d, *J*=8.1 Hz, 1H), 5.87 (s, 2H), 3.58 (s, 2H), 2.86 (t, 2H), 2.76- 2.64 (m, 2H), 2.53 (m, 1H), 2.31-2.24 (m, 2H), 2.14 (m, 4H), 1.60-1.22 (m, 5H), 1.21-1.15 (m, 2H).
EI-MS *m*/*z* (%) 394 (M⁺, 1.5), 277 (100), 98 (95.8), 91 (93.2);
HR-MS [M + H]⁺ observed = 394.2228, estimated = 394.2256.

### Example 3

### Compound I-a-c:

### 1-benzyl-3-(benzo[1,3]dioxol-5-yl)-4-(morpholinomethyl)pyrrolidin-3-ol

Compound **I-a-c** was prepared by following the procedure described for the synthesis of compound **I-a-a** via replacement of 4-phenylpiperidine by morpholine.
IR (film) 3370, 2926, 1504, 1487 cm⁻¹;
¹H NMR (CDC1₃, 300 MHz) δ 7.34-7.15 (m, 5H), 7.09 (d, J=1.5 Hz, 1H), 7.02 (dd, *J* = 7.8 Hz and J=1.5 Hz, 1H), 6.68 (d, *J* = 7.8 Hz, 1H), 5.87 (s, 2H), 3.58 (s, 2H), 3.56-3.49 (m, 4H), 2.88-2.83 (m, 2H), 2.78-2.71 (m, 2H), 2.60-2.50 (m, 1H), 2.35-2.29 (m, 2H), 2.20- 2.15 (m, 4H), 1.53-1.47 (m, 1H).
EI-MS *m*/*z* (%) 396 (M⁺, 7.1), 378 (1.4), 278 (51.7), 1656 (100), 91 (78.7);
HR-MS [M + H] + observed = 396.2006, estimated = 396.2049.

### Example 4

### Compound I-a-d

### 1-benzyl-3-(benzo[1,3]dioxol-5-yl)-4-[(diethylamino)methyl]pyrrolidin-3-ol

Compound **I-a-d** was prepared by following the procedure described for the synthesis of compound **I-a-a** via replacement of 4-phenylpiperidine by diethylamine.
IR (film) 3314,2931,1665,1487,1452 cm⁻¹;
¹H NMR (CDC1₃, 300 MHz) δ 7.31-7.17 (m, 5H), 7.11-7.02 (m, 2H), 6.70-6.67 (m, 1H), 5.87 (s, 2H), 3.64-3.58 (m, 3H), 2.86-2.76 (m, 4H), 2.46-2.35 (m, 4H), 2.21-2.14 (m, 2H), 1.58-1.49 (m, 1H), 1.28-1.13 (m, 6H);
EI-MS *m*/*z* (%) 382 (M⁺, 2.1), 91 (77.3), 56 (100);
HR-MS [M + H] ⁺observed = 382.2296, estimated = 382.2256.

### Example 5

### Compound I-b-a

### 1-benzyl-3-phenyl-4-[(4-phenylpiperidin-1-yl)methyl]pyrrolidin-3-ol

Compound **I-b-a** was prepared by following the procedure described for the synthesis of compound **I-a-a** via replacement of ethyl 2-(benzo [1,3]dioxol-5-yl)-2-oxoacetate by ethyl benzoylformate.
¹H NMR (CDCl₃, 300M Hz) δ 7.69-7.61 (m, 2H), 7.37-7.13 (m, 13H), 3.67 (s, 2H), 3.01-2.81 (m, 4H), 2.65 (m, 1H), 2.57-2.52 (m, 1H), 2.46-2.35 (m, 2H), 2.08-2.02 (m, 2H), 1.80-1.69 (m, 4H), 1.51-1.41 (m, 2H);
EI-MS *m*/*z* (%) 427 (M⁺ + 1, 0.79), 336 (3.81), 293 (43.34), 252 (19.54), 233 (95.09), 200 (14.97), 174 (97.91), 91 (100);
HR-MS [M + H]⁺ observed = 426.2657, estimated = 426.2671.

### Example 6

### Compound I-b-b

### (3-hydroxy-3-phenyl-4-[(4-phenylpiperidin-1-yl)methyl]pyrrolidin-1-yl)(phenyl)methanone

According to scheme II, compound **I-b-a** (0.4 g) was dissolved in methanol (30 ml) and Pd/C (50 mg) was added to the solution. The mixture was stirred at room temperature for 12 h under 1 atm hydrogen. The palladium on carbon was filtered off and methanol was evaporated by rotatory evaporator. 96mg of the residue was taken and dissolved in dry dichloromethane (2 ml) and triethylamine (0.059 ml) was added to the solution under N₂ atmoshphere. Then the solution of benzoyl chloride (0.04 ml) in dichloromethane (1 ml) was added dropwise to the mixture at 0 °C. The reaction mixture was warmed to room temperature and reacted for 4 h. Then water was added and the two layers were separated. The organic layer was washed with brine, dried, filtered, concentrated and chromatographed (gradient elution 1/2 EtOAc/hexane to 1/1/0.5 EtOAc/hexane/ Et₃N) to give a solid product (65 mg, 52%).
IR (KBr) 3269, 3028, 2922, 2808, 1723, 1592, 1571, 1453, 1381, 1247 cm⁻¹;
¹H NMR (CDCl₃, 300 MHz), δ 7.61-6.91 (m, 15H), 4.14-3.92 (m, 2H), 3.79-3.64 (m, 2H), 3.18-3.18 (m, 1H), 3.05-2.96 (m, 1H), 2.87-2.78 (m, 2H), 2.65-2.59 (m, 1H), 2.53-2.43 (m, 2H), 2.29-2.05 (m, 2H), 1.88-1.73 (m, 4H);
EI-MS *m*/*z* (%) 440 (M⁺, 0.93), 401 (5.42), 292 (16.12), 200 (12.45), 186 (35.38), 174 (100), 160 (7.34);
HR-MS [M + H]⁺ observed = 440.2448, estimated = 440.2463.

### Example 7

### Compound I-b-c

### (3-hydroxy-3-phenyl-4-[(4-phenylpiperidin-1-yl)methyl]pyrrolidin-1-yl)(2-iodophenyl) methanone

Compound **I-b-c** was prepared by following the procedure described for the synthesis of compound **I-b-b** via replacement of benzoyl chloride by 2-iodobenzoyl chloride.
IR (KBr) 3027, 2933, 2808, 1732, 1634, 1440, 1425, 1248, 762, 699 cm⁻¹;
¹H NMR (CDC1₃ 300 MHz) δ 7.89-7.79 (m, 1H), 7.62-7.59 (m, 1H), 7.54-7.46 (m, 1H), 7.46-7.17 (m, 11H), 4.10-4.02 (m, 1H), 3.97-3.93 (m, 1H), 3.59 (m, 1H), 3.40 (m, 1H), 3.14 (m, 1H), 3.00 (m, 1H), 2.85-2.79 (m, 2H), 2.63-2.44 (m, 3H), 2.29-2.04 (m, 2H), 1.89-1.74 (m, 4H);
EI-MS *m*/*z* (%) 566 (M⁺, 0.37), 565 (0.40), 406 (0.68), 355 (0.5), 231 (8.77), 174 (100), 160 (3.43), 105 (5.04), 91 (3.57).

### Example 8

### Compound I-b-d

### 3-phenyl-4-[(4-phenylpiperidin-1-yl)methyl]-1-(phenylsulfonyl)pyrrolidin-3-ol

Compound I-b-d was prepared by following the procedure described for the synthesis of compound I-b-b via replacement of benzoyl chloride by benzenesulfonyl chloride.
IR (KBr) 3496, 3062, 2933, 2812, 1737, 1494, 1447, 1345, 1247, 1168 cm⁻¹;
¹H NMR (CDC1₃, 300 MHz) δ 7.83 (d, *J =* 8.7 Hz, 2H), 7.57-7.47 (m, 3H), 7.34-7.07 (m, 10H), 3.64 (d, *J_{AB} =* 10.8 Hz, 1 H), 3.60-3.58 (m, 1 H), 3.43 (d, *J_{AB} =* 10.8 Hz, 1 H), 3.32 -3.27 (q, 1 H), 2.76 (d, 1H), 2.68 (d, 1H), 2.42-2.30 (m, 4H), 2.07-1.94 (m, 2H), 1.74-1.59 (m, 4H);
EI-MS *m*/*z* (%) 477 (M⁺ + 1, 0.33), 336 (25.49), 335 (100), 174 (83.55), 160 (3.01).

### Example 9

### Compound I-b-e

### Cyclopentyl-(3-hydroxy-3-phenyl-4-[(4-phenylpiperidin-1-yl)methyl]pyrrolidin-1-yl)metha none

Compound **I-b-e** was prepared by following the procedure described for the synthesis of compound **I-b-b** via replacement of benzoyl chloride by cyclopentanecarbonyl chloride.
IR (KBr) 3276, 2953, 1608, 1493, 1453, 1381 cm-¹;
¹H NMR (CDCl₃, 300 MHZ) δ 7.58 (m, 2H), 7.39 (m, 2H), 7,31 (m, 2H), 7.20 (m, 2H), 3.90-3.70 (m, 4H), 3.14-3.05 (m, 1H), 2.88-2.69 (m, 3H), 2.60-2.44 (m, 3H), 2.29-2.14 (m, 2H), 1.91-1.71 (m, 10H), 1.62-1.54 (m, 2H);
EI-MS *m*/*z* (%) 432 (M⁺, 0.56), 292 (2.48), 174 (100);
HR-MS [M + H]⁺ observed = 432.2800, estimated = 432.2776.

### Example 10

### Compound I-b-f

### Cyclohexyl-(3-hydroxy-3-phenyl-4-[(4-phenylpiperidin-1-yl)methyl]pyrrolidin-1-yl)methan one

Compound **I-b-f** was prepared by following the procedure described for the synthesis of compound **I-b-b** via replacement of benzoyl chloride by cyclohexanecarbonyl chloride.
IR (KBr) 3253, 2939, 2850, 1606, 1493, 1453, 1398 cm⁻¹;
¹H NMR (CDC1₃, 300 MHZ) δ 7.57-7.51 (m, 2H), 7.43-7.20 (m, 8H),3.89-
3.70 (m, 4H), 3.10 (t, 1H), 2.85-2.71 (m, 2H), 2.58-36 (m, 3H), 2.32-2.06 (m, 3H), 1.82-1.43 (m, 13H);
EI-MS *m*/*z* (%) 446 (M⁺, 0.55), 174 (100);
HR-MS [M + H]⁺ observed = 446.2945, estimated = 446.2933.

### Example 11

### Compound I-b-g

### 1-benzyl-3-phenyl-4-{[4-(3-phenylpropyl)piperidin-1-yl]methyl}]pyrrolidin-3-ol

Compound **I-b-g** was prepared by following the procedure described for the synthesis of compound **I-b-a** via replacement of 4-phenylpiperidine by 4-(3-phenylpropyl)piperidine.
IR (KBr) 3200, 3027, 2928, 2806, 1739, 1494, 1474, 1446, 1376 cm⁻¹;
¹H NMR (CDC1₃, 300 MHZ) δ 7.78-7.60 (m, 2H), 7.41-7.14 (m, 13H), 3.70 (m, 2H), 3.01-2.53 (m, 8H), 2.58-2.53 (m, 2H), 2.45-2.41 (m, 2H), 2.00-1.83 (m, 1H), 1.80-1.50 (m, 4H), 1.43-1.18 (m, 5H);
EI-MS *m*/*z* (%) 377 (3.08), 334 (30.38), 233 (79.19), 216 (76.27), 91 (100).

### Example 12

### Compound I-b-h

### 1-[(1-benzyl-4-hydroxy-4-phenylpyrrolidin-3-yl)methyl]-4-(3-phenylpropyl)piperidin-4-ol

Compound **I-b-h** was prepared by following the procedure described for the synthesis of compound **I-b-a** via replacement of 4-phenylpiperidine by 4-(3-phenylpropyl)piperidin-4-ol.
¹H NMR (CDCl₃, 300 MHZ) δ 7.54-7.51 (m, 2H), 7.32-7.08 (m, 13H), 3.61-3.57 (m, 3H), 2.96 (m, 2H), 2.84 (d, 1H), 2.73 (q, 1H), 2.63-2.48 (m, 4H), 2.40-2.18 (m, 3H), 2.12-1.97 (m, 1H), 1.62-1.32 (m, 7H), 1.26-1.15 (m, 2H);
EI-MS *m*/*z* (%) 393 (4.19), 350 (31.62), 233 (83.79), 91 (100);
HR-MS [M + H]⁺ observed = 485.3162, estimated = 485.3162.

### Example 13

### Compound I-b-j

### 1-benzyl-3-(4-fluorophenyl)-4-((4-phenylpiperidin-1-yl)methyl)pyrrolidin-3-ol

Compound **I-b-j** was prepared by following the procedure described for the synthesis of compound **I-b-a** via replacement of ethyl 2-oxo-2-phenylacetate by ethyl 2-(4-fluorophenyl)-2-oxoacetate.
¹H NMR (CDC1₃, 300MHz) δ 7.62-7.55 (m, 2H), 7.37-7.08 (m, 12H), 3.6 (s, 2H), 2.99-2.75 (m, 5H), 2.64-2.45 (m, 2H), 2.43-2.33 (m, 3H), 2.03 (m, 1H), 1.80-1.42 (m, 5H);
EI-MS *m*/*z* (%) 353 (1.64), 335 (1.81), 310 (25.31), 292 (24.04), 252 (38.22), 234 (38.90), 174 (100), 91 (54.63).

### Example 14

### Compound I-b-k

### 1-{[1-benzyl-4-(4-fluorophenyl)-4-hydroxypyrrolidin-3-yl]methyl}-4-(3-phenylpropyl)piper idin-4-ol

Compound **I-b-k** was prepared by following the procedure described for the synthesis of compound **I-b-h** via replacement of ethyl 2-oxo-2-phenylacetate by ethyl 2-(4-fluorophenyl)-2-oxoacetate.
¹H NMR (CDCl₃, 300MHz) δ 7.63-7.57 (m, 2H), 7.38-7.16 (, 10H), 7.02-6.96 (m, 2H), 3.73-3.66 (m, 3H), 2.96-2.89 (m, 2H), 2.89-2.81 (m, 2H), 2.63-2.54 (m, 4H), 2.43-2.18 (m, 4H), 1.71-1.64 (m, 3H), 1.61-1.42 (m, 4H), 1.32-1.24 (m, 2H);
EI-MS *m*/*z* (%) 283 (3.31), 268 (5.14), 258 (18.72), 232 (52.00), 91 (100);

### Example 15

### Compound I-c-a

### 1-benzyl-3-methyl-4-[(4-phenylpiperidin-1-yl)methyl]pyrrolidin-3-ol

Compound **I-c-a** was prepared by following the procedure described for the synthesis of compound **I-b-a** via replacement of ethyl 2-oxo-2-phenylacetate by ethyl acetonate (0.088 g, 30.2%).
¹H NMR (CDC1₃, 300MHz) δ 7.20-7.06 (m, 10H), 3.73 (t, *J* = 5.4 Hz, 1H), 3.58-3.45 (m, 2H), 3.16 (d, 1H), 2.89 (d, 1H), 2,75-2.31 (m, 5H), 2.18-2.00 (m, 2H), 1.90 (m, 1H), 1.78-1.54 (m, 4H), 1.26 (s, 3H);
EI-MS *m*/*z* (%) 274 (1.47), 273 (7.49), 172 (100), 160 (16.83), 91 (94.57).

### Example 16

### Compound I-c-b

### 1-benzyl-3-methyl-4-[(4-phenylpiperidin-1-yl)methyl]pyrrolidin-3-yl benzoate

The compound **I-c-a** (1eq) obtained above was dissolved in CH₂Cl₂ and triethylamine (1.5 eq) was added.. Benzoyl chloride (1.2 eq) was added at 0°C. Then the reaction mixture was stirred at room temperature for 6 h and quenched with water. The aqueous layer was extracted with CH₂Cl₂ and the combined organic layer was washed with brine, dried, chromatographed to give compound **I-c-b**.
¹H NMR (CDCl₃, 300MHz) δ 7.95-7.87 (m, 2H), 7.46-7.08 (m, 13H), 4.26 (t, *J*= 6.1 Hz, 1H), 3.65-3.51 (m, 3H), 3.32 (d, 1H), 3.08-2.83 (m, 5H), 2.78-2.56 (m, 3H), 2.42-2.20 (m, 3H), 2.09 (m, 1H), 1.94 (m, 1H), 1.65 (s, 3H);
EI-MS *m*/*z* (%) 377 (1.45), 172 (100), 91 (73.73).

### Example 17

### Compound I-d-a

### 1-benzyl-4-[(4-phenylpiperidin-1-yl)methyl]pyrrolidin-3-ol

Compound **I-d-a** was prepared by following the procedure described for the synthesis of compound **I-b-a** via replacement of ethyl 2-oxo-2-phenylacetate by ethyl glyoxylate.
¹H NMR (CDCl₃, 300MHz) δ 7.33-7.19 (m, 10H), 3.70 (d, *J* = 12.9 Hz, 1H), 3.62 (d, *J* = 12.9 Hz, 1H), 3.20 (d, 1H), 3.04-2.70 (m, 6H), 2.56-2.25 (m, 4H), 2.23-2.13 (m, 2H), 1.90-1.76 (m, 4H);
EI-MS *m*/*z* (%) 332 (0.54), 259 (13.15), 174 (57.86), 91 (100).

### Example 18

### Compound I-d-b

### 1-[(1-benzyl-4-phenoxypyrrolidin-3-yl)methyl]-4-phenylpiperidine

According to scheme VI, the compound **I-b-a** (1 eq) was dissolved in CH₂Cl₂ and triethylamine (1.5 eq) was added to it. Methanesulfonyl chloride (1.2 eq) was added at 0 °C. Then the reaction mixture was stirred at the same temperature for 0.5 h and washed with water and brine respectively. The organic layer was dried and concentrated by rotatory evaporator to give a white solid.

The white solid was dissolved in THF and sodium phenolate (2 eq) was added. The reaction mixture was refluxed for 6 h and water was added. Then the organic layer was dried, and chromatographed to give compound **I-d-b.**
¹H NMR (CDCl₃, 300MHz) δ 7.35-7.17 (m, 10H), 6.98-6.89 (m, 3H), 6.84-6.80 (m, 2H), 4.19-4.15 (m, 1H), 3.99 (t, 1H), 3.80 (d, *J* = 13.2 Hz, 1H), 3.73 (d, *J* = 13,2 Hz, 1H), 3.18-3.12 (m, 2H), 2.95-2.83 (m, 5H), 2.79-2.62 (m, 2H), 2.50 (m, 1H), 2.06-1.99 (m, 2H), 1.80-1.73 (m, 4H);
EI-MS *m*/*z* (%) 426 (M⁺+1, 1.00), 335 (17.96), 266 (18.06), 94 (100), 91 (57.79).

### Example 19

### Compound I-d-c

### 1-{[1-benzyl-4-(thiophenyl)pyrrolidin-3-yl]methyl}-4-phenylpiperidine

Compound **I-d-c** was prepared by following the procedure described for the synthesis of compound **I-d-b** via replacement of sodium phenolate by sodium benzenethiolate.
¹H NMR (CDCl₃, 300MHz) δ 7.40-7.16 (m, 15H), 3.75 (dd, *J_{AB} =* 13.2 Hz, 2H), 3.33 (m, 1H), 2.94-2.70 (m, 8H), 2.68 (m, 1H), 2.59 (m, 1H), 2.01 (m, 1H), 1.90 (m, 1H), 1.78-1.60 (m, 4H);
EI-MS *m*/*z* (%) 442 (M⁺+1, 3.83), 351 (21.14), 333 (100), 174 (30.73), 91 (83.42).

### Example 20

### Compound I-d-d

### 1-{[1-benzyl-4-(phenylsulfonyl)pyrrolidin-3-yl]methyl}-4-phenylpiperidine

Compound **I-d-c** (1 eq) was dissolved in CH₂Cl₂, and mCPBA (2.0 eq) was added. Then the reaction mixture was stirred at room temperature overnight and extracted with water. The organic layer was dried and chromatographed to give compound **I-d-d**.
¹H NMR (CDCl₃, 300MHz) δ 7.84-7.81 (m, 2H), 7.58-7.21 (m, 13H), 4.80-4.68 (m, 1H), 4.53 (dd, *J_{AB}* = 13.2 Hz, 2H), 4.20-4.11 (m, 2H), 4.08-3.96 (m, 1H), 3.64-3.51 (m, 3H), 3.30-3.12 (m, 3H), 2.98-2.84 (m, 1H), 2.59 (m, 3H), 1.70 (m, 2H);
EI-MS *m*/*z* (%) 382 (12.12), 332 (8.52), 282 (27.51), 267 (33.19), 91 (100).

### Example 21

### Compound I-d-e

### 1-benzyl-N-phenyl-4-[(4-phenylpiperidin-1-yl)methyl]pyrrolidin-3-amine

Compound **I-d-e** was prepared by following the procedure described for the synthesis of compound **I-d-b** via replacement of sodium phenolate by aniline.
¹H NMR (CDCl₃, 300MHz) δ 7.33-7.20 (m, 15H), 4.07 (q, 1H), 3.64 (dd, *J_{AB} =* 13.2 Hz, 2H), 3.17 (d, 1H), 3.07-2.91 (m, 2H), 2.75-2.72 (m, 2H), 2.56-2.40 (m, 2H), 2.40-2.25 (m, 1H), 2.18 (m, 2H), 2.00 (m, 2H), 1.90-1.65 (m, 4H);
ESI-MS *m*/*z* 426 (M⁺+1).

### Example 22

### Compound II-a-a

### Benzyl 1-[(1-benzyl-4-hydroxy-4-phenylpyrrolidin-3-yl)methyl]piperidin-4-yl (ethyl)carbamate

According to scheme III, the intermediate for preparing compound **I-b-a**, 1-benzyl-4-hydroxy-5-oxo-4-phenylpyrrolidine-3-carboxylic acid (1 eq), and DCC (1.1 eq) and HOSu (1.1 eq) were dissolved in THF. The resulting mixture was stirred at room temperature for 6h. The mixture was filtered. Then N-(piperidin-4-yl)acetamide was added to the filtrate and the reaction mixture was stirred at room temperature for another 6 h. The solvent was evaporated and the residue was extracted with ethyl acetate and water and the organic layer was washed with brine, dried, filtered, and chromatographed to give a solid compound.

The solid compound was dissolved in THF, and then LiAlH₄ (8 eq) was added and the mixture was heated under reflux for 24 h. The solution was quenched with 10% NaOH solution. The mixture was filtered and the solvent was evaporated to afford a sticky compound.

The sticky compound was dissolved in CH₂Cl₂, and triethylamine (2 eq) and benzyl chloroformate (1.5 eq) was added. Then the reaction mixture was reacted for 3 h and extracted with water. The organic layer was washed with brine, dried, filtered, and chromatographed to give compound **II-a-a**.
¹H NMR (CDC1₃, 300 MHz) δ 7.38-7.12 (m, 15H), 5.07 (m, 2H), 3.91-3.89 (m, 1H), 3.87-3.33 (m, 2H), 3.20-3.07 (m, 3H), 2.95-2.85 (m, 3H), 2.82-2.72 (m, 2H), 2.62-2.56 (m, 1H), 2.45-2.40 (m, 2H), 2.36-2.29 (m, 1H), 2.04-1.98 (m, 2H), 1.39-1.12 (m, 3H), 1.06 (*t, J =* 7.2 Hz, 3H);
EI-MS *m*/*z* (%) 528 (M⁺ + H⁺, 1.43), 484 (2.56), 395 (61.84), 394 (67.91), 259 (16.15), 234 (58.02), 141 (48.31), 98 (40.18), 91 (100).

### Example 23

### Compound II-a-b

### Benzyl 1-[(1-benzyl-4-(4-fluorophenyl)-4-hydroxypyrrolidin-3-yl)methyl]piperidin-4-yl (ethyl)carbamate

Compound **II-a-b** was prepared by following the procedure described for the synthesis of compound **II-a-a** via replacement of 1-benzyl-4-hydroxy-5-oxo-4-phenylpyrrolidine-3-carboxylic acid by 1-benzyl-4-(4-fluorophenyl)-4-hydroxy-5-oxopyrrolidine-3-carboxylic acid.
¹H NMR (CDC1₃, 300MHz) δ 7.60 (m, 2H), 7.36-7.28 (m, 11H), 6.98 (t, 2H), 5.11 (s, 2H), 3.76-3.60 (m, 2H), 3.10 (s, 2H), 3.00-2.87 (m, 2H), 2.87-2.71 (m, 2H), 2.63-2.30 (m, 3H), 2.05 (m, 2H), 1.73 (m, 3H), 1.40-1.20 (m, 2H), 1.11 (m, 2H), 0.85 (t, 3H);
ESI-MS *m*/*z* 546 (M⁺+1).

### Example 24

### Compound II-a-c:

### Benzyl 1-[(1-benzyl-4-hydroxy-4-methylpyrrolidin-3-yl)methyl]piperidin-4-yl (ethyl)carbamate

Compound **II-a-c** was prepared by following the procedure described for the synthesis of compound **II-a-a** via replacement of 1-benzyl-4-hydroxy-5-oxo-4-phenylpyrrolidine-3-carboxylic acid by 1-benzyl-4-hydroxy-4-methyl-5-oxopyrrolidine-3-earboxylic acid.
¹H NMR (CDC1₃, 300MHz) δ 7.38-7.27 (m, 10H), 5.14 (s, 2H), 4.20 (t, *J* = 4.2 Hz, 1H), 3.58 (dd, *J_{AB}* = 13.2 Hz), 3.20 (d, 2H), 2.98 (d, 1H), 2.80-2.65 (m, 3H), 2.55 (d, 1H), 2.40-1.84 (m, 5H), 1.80-1.55 (m, 4H), 1.42-1.05 (m, 4H), 0.88 (t, 3H);
ESI-MS *m*/*z* 466 (M⁺+1).

### Example 25

### Compound III-a-a

### 4-nitrobenzyl allyl(1-((1-benzyl-4-hydroxy-4-phenylpyrrolidin-3-yl)methyl)piperidin-4-yl) carbamate

According to scheme IV, the intermediate for preparing compound **I-b-a,** 1-benzyl-4-hydroxy-5-oxo-4-phenylpyrrolidine-3-carboxylic acid (1 eq), and DCC (1.1 eq) and HOSu (1.1 eq) were dissolved in THF. The resulting mixture was stirred at room temperature for 6h. The mixture was filtered. Then triethylamine (2 eq) and piperidine-4,4-diol hydrochloride(1.1 eq) were added to the filtrate and the reaction mixture was stirred overnight. THF was evaporated and the residue was extracted with ethyl acetate and water and the organic layer was washed with brine, dried, filtered and concentrated to give a white solid compound.

The solid was dissolved in THF and LiAlH₄ (8 eq) was added, and the mixture was heated under reflux for 24 h. The solution was quenched with 10% NaOH. The mixture was filtered and concentrated to afford a solid foam secondary alcohol.

The solid foam was dissolved in CH₂Cl₂ (1 eq) and triethylamine (1.5 eq) and 4-nitrobenzyl chloroformate (1.2 eq) were added. Then the reaction mixture was reacted at room temperature for 4 h and extracted with water. The organic layer was dried and chromatographed to give compound III-a-a.
IR (kBr) 2938, 2806, 1751, 1710, 1608, 1523, 1496, 1448, 1378, 1348, 1261 cm⁻¹;
¹H NMR (CDC1₃, 300MHz) δ 8.27-8.22 (m, 2H), 7.55-7.25 (m, 12H), 5.97-5.82 (m, 1H), 5.36-5.07 (m, 4H), 4.39 (m, 1H), 3.68 (m, 2H), 3.25 (m, 2H), 2.80-2.22 (m, 6H), 1.79 (m, 4H);
ESI-MS *m*/*z* 585 (M⁺+1);
HR-MS [M + H]⁺observed = 585.3070, estimated = 585.3071.

### Example 26

### Compound III-a-b

### 4-nitrobenzyl allyl(1-((1-benzoyl-4-hydroxy-4-phenylpyrrolidin-3-yl)methyl)piperidin-4-yl) carbamate

According to scheme V, the intermediate for preparing compound **III-a-a,** the secondary alcohol (1 eq), was dissolved in CH₂Cl₂, and a catalytic amount of DMAP (0.1 eq), TEA (5 eq) and acetic anhydride (3 eq) were added to it. The resulting mixture was stirred at 0 °C for 2h. The mixture was extracted with CH₂Cl₂ and water and the organic layer was washed with brine, dried and chromatographed on silica gel to give a foam solid.

The above foam solid was dissolved in methanol and Pd/C (5 %) was added. The mixture was hydrogenated under 1 atm hydrogen overnight. Then the catalyst was filtered off and the solvent of the filtrate was concentrated to give a foam compound. The foam compound was dissolved in CH₂Cl₂ and triethylamine (1.5 eq) and benzoyl chloride (1.2 eq) were added. Then the reaction mixture was stirred for 2 h and extracted with water. The organic layer was washed with brine, dried and chromatographed to give a foam compound.

The foam compound (1 eq) was dissolved in methanol and water (v: v = 5: 1) and potassium carbonate (2 eq) was added. The reaction mixture was stirred for 4 h followed by removing methanol and extracted with ethyl acetate twice. The combined organic layer was washed with brine, dried, filtered and concentrated to give a white foam compound.

A solution of oxalyl chloride (1.3 eq) in CH₂Cl₂ was added dropwise to a solution of DMSO in CH₂Cl₂ at -78 °C. The reaction mixture was stirred for another 10 min. A solution of the above white foam compound (1 eq) in CH₂Cl₂ was added and stirring was continued for an additional 30 min. TEA (3 eq) was added and the reaction mixture was allowed to warm to room temperature. Water and EtOAc were then added and shaken to separate the two phases. The organic phase was washed with brine, dried and chromatographed on silica gel to give its keto derivative.

The mixture of the keto derivative (1 eq) and sodium triacetoxyborohydride (1.5 eq) was dissolved in 1,2-dichloroethane. Allyl amine (1 eq) and acetic acid (1 eq) were added and the reaction mixture was stirred overnight. Then to the mixture was added saturated sodium bicarbonate solution and the mixture was extracted with ethyl acetate twice. The combined organic layer was washed with brine, dried, filtered and concentrated to give white foam.

The white foam (1 eq) was dissolved in CH₂Cl₂ and triethylamine (2 eq) and 4-nitrobenzyl chloroformate (1.5 eq) were added. Then the reaction mixture was stirred for 2 h. Water and EtOAc were then added and shaken to separate the two phases. The organic layer was washed with brine, dried, filtered and chromatographed to give compound **III-a-b.**
IR (KBr) 3375, 3063, 2943, 1701, 1627, 1608, 1577, 1522, 1496, 1421, 1346, 1250 cm⁻¹;
¹H NMR (CDCl₃, 300MHz) δ 8.24-8.19 (m, 2H), 7.61-7.21 (m, 12H), 5.82-5.77 (m, 1H), 5.22-5.11 (m, 4H), 4.14-3.61 (m, 8H), 3.17-3.07 (m, 1H), 2.98-2.40 (m, 4H), 2.20 (m, 1H), 1.82-1.50 (m, 4H);
ESI-MS *m*/*z* 599 (M⁺+1);
HR-MS [M + H]⁺ observed = 599.2879, estimated = 599.2864.

### Example 27

### Compound III-a-c

### 4-nitrobenzyl allyl(1-((4-hydroxy-1-(2-iodobenzoyl)-4-phenylpyrrolidin-3-yl) methyl)piperidin-4-yl)carbamate

Compound **III-a-c** was prepared by following the procedure described for the synthesis of compound **III-a-b** via replacement of benzoyl chloride by 2-iodobenzoyl chloride.
IR (KBr) 3375, 2941, 1701, 1637, 1522, 1467, 1421, 1345, 1249 cm⁻¹;
¹H NMR (CDCl₃, 300MHz) δ 8.24-8.20 (m, 2H), 7.86-7.80 (m, 1H), 7.55-7.06 (m, 10H), 5.95-5.76 (m, 1H), 5.23-5.12 (m, 4H), 4.16-4.04 (m, 2H), 3.96-3.85 (m, 3H), 3.70-3.13 (m, 3H), 3.03 (m, 1H), 2.80 (m, 2H), 2.57 (m, 1H), 2.18-2.02 (m, 2H), 1.87-1.64 (m, 4H);
ESI-MS *m*/*z* 725 (M⁺+1);
HR-MS [M + H]⁺ observed = 725.1833, estimated = 725.1831.

### Example 28

### Compound III-a-d

### 4-nitrobenzyl 1-((1-(1-naphthoyl)-4-hydroxy-4-phenylpyrrolidin-3-yl)methyl)piperidin-4-yl(allyl)carbamate

Compound **III-a-d** was prepared by following the procedure described for the synthesis of compound **III-a-b** via replacement of benzoyl chloride by 1-naphthoyl chloride.
IR (KBr) 3381, 3060, 2943, 1701, 1633, 1522, 1465, 1428, 1384, 1346, 1249 cm⁻¹;
¹H NMR (CDC1₃, 300MHz) δ 8.23-8.18 (m, 2H), 7.96-7.82 (m, 3H), 7.58-7.21 (m, 11H), 5.95-5.76 (m, 1H), 5.21-5.11 (m, 4H), 4.20-3.96 (m, 3H), 3.94-3.58 (m, 3H), 3.52-3.21 (m, 2H), 3.10 (m, 1H), 2.82-2.52 (m, 4H), 2.25-2.06 (m, 1H), 1.74-1.39 (m, 4H);
ESI-MS *m*/*z* 649 (M⁺+1);
HR-MS [M + H]⁺ observed = 649.3016, estimated = 649.3021.

### Example 29

### Compound III-a-e

### 4-nitrobenzyl allyl(1-((1-(cyclopentanecarbonyl)-4-hydroxy-4-phenylpyrrolidin-3-yl) methyl)piperidin-4-yl)carbamate

Compound **III-a-e** was prepared by following the procedure described for the synthesis of compound **III-a-b** via replacement of benzoyl chloride by cyclopentanecarbonyl chloride.
IR (KBr) 3375, 2948, 1700, 1637, 1523, 1467, 1345, 1249 cm⁻¹;
¹H NMR (CDCl₃, 300MHz) δ 8.23 (d, 2H), 7.54-7.23 (m, 8H), 5.83-5.77 (m, 1H), 5.23-5.12 (m, 4H), 4.10-3.67 (m, 7H), 3.58-3.40 (m, 1H), 3.06-2.60 (m, 4H), 2.59-2.40 (m, 1H), 2.14-2.03 (m, 1H), 2.00-1.48 (m, 13H);
ESI-MS *m*/*z* 591 (M⁺+1);
HR-MS [M + H]⁺ observed = 591.3168, estimated = 591.3177.

### Example 30

### Compound III-a-f

### 4-nitrobenzyl allyl(1-((1-(cyclohexanecarbonyl)-4-hydroxy-4-phenylpyrrolidin-3-yl)methyl) piperidin-4-yl)carbamate

Compound **III-a-f** was prepared by following the procedure described for the synthesis of compound **III-a-b** via replacement of benzoyl chloride by cyclohexanecarbonyl chloride.
IR (KBr) 3375, 2933, 2855, 1701, 1638, 1523, 1450, 1346, 1250 cm⁻¹;
¹H NMR (CDCl₃, 300MHz) δ 8.22 (d, *J* = 7.8 Hz), 7.60-7.52 (m, 3H), 7.42-7.11 (m, 4H), 5.92-5.76 (m, 1H), 5.22 (s, 2H), 5.16-5.10 (m, 2H), 4.15-3.62 (m, 7H), 3.58-3.37 (m, 1H), 3.11-2.60 (m, 4H), 2.58-2.00 (m, 2H), 2.00-1.44 (m, 15H);
ESI-MS *m*/*z* 605 (M⁺+1);
HR-MS [M + H]⁺ observed = 605.3333, estimated = 605.3334.

### Example 31

### Compound III-a-g

### 4-nitrobenzyl 1-[(1-benzyl-4-(4-fluorophenyl)-4-hydroxypyrrolidin-3-yl)methyl]piperidin-4-yl(allyl)carbamate

Compound **III-a-g** was prepared by following the procedure described for the synthesis of compound **III-a-a** via replacement of 1-benzyl-4-hydroxy-5-oxo-4-phenylpyrrolidine-3-carboxylic acid by 1-benzyl-4-(4-fluorophenyl)-4-hydroxy-5-oxopyrrolidine-3-carboxylic acid.
¹H NMR (CDC1₃, 300MHz) δ 8.21 (m, 2H), 7.64-7.47 (m, 3H), 7.40-7.20 (m, 6H), 7.05-6.96 (m, 2H), 5.89 (m, 1H), 5.18-5.06 (m, 4H), 3.68 (m, 3H), 3.28-3.21 (m, 3H), 2.95 (d, 1H), 2.86-2.74 (m, 3H), 2.60 (m, 1H), 2.53-2.25 (m, 5H), 1.85-1.75 (m, 4H).
ESI-MS *m*/*z* (M⁺+1) 603;
HR-MS [M + H]⁺ observed = 625.2808, estimated = 625.2797.

### Example 32

### Compound I-c-c

Compound **I-c-c** was prepared by following the procedure described for the synthesis of compound **I-c-b** via replacement of ethyl acetonate by ethyl glyoxylate (0.086 g,29%).

### Example 33

### Compound IV-a-a and compound IV-a-b

Compound **IV-a-a** and compound **IV-a-b** were prepared by following the procedure described for the synthesis of compound **I-b-a** via replacement of 3-aminopropanoic acid by 3-aminobutanoic acid (0.020 g, 31%).

### Example 34

### Compound III-a-h

Compound **III-a-h** was prepared by following the procedure described for the synthesis of compound **III-a-e** via replacement of 4-nitrobenzyl chloroformate by benzyl chloroformate.
¹H NMR (CDCl₃, 300MHz) 7.52-7.26 (m, 10H), 5.85-5.77 (m, 1H), 5.21-5.08 (m, 4H), 4.05-3.88 (m, 1H) 3.87-3.64 (m, 6H), 3.01-2.39 (m, 7H), 2.38-2.14 (m, 1H), 1.87-1.48 (m, 12H);
ESI-MS *m*/*z* 546.4 (M⁺+1).

### Example 35

### Compound III-a-i

Compound **III-a-i** was prepared by following the procedure described for the synthesis of compound **III-a-e** via replacement of 4-nitrobenzyl chloroformate by 4-methoxybenzyl chloroformate.
¹H NMR (CDC1₃, 300MHz) δ 7.52-7.24 (m, 7H), 6.95-6.82 (d, 2H), 5.92-5.68 (m, 1H), 5.26-5.02 (m, 4H), 4.21-3.63 (m, 10H), 3.22-2.34 (m, 7H), 2.02-1.51 (m, 13H).

### Example 36

### Compound III-a-j

Compound **III-a-j** was prepared by following the procedure described for the synthesis of compound **III-a-e** via replacement of 4-nitrobenzyl chloroformate by 4-bromobenzyl chloroformate.
¹H NMR (CDCl₃, 300MHz) δ 7.55-7.15 (m, 9H), 5.86-5.67 (m, 1H), 5.30-5.00 (m, 4H), 4.17-3.62 (m, 7H), 3.06-2.35 (m, 6H), 2.22-2.02 (m, 2H), 1.86-1.53 (m, 12H).

### Example 37

### Compound III-a-k

Compound **III-a-k** was prepared by following the procedure described for the synthesis of compound **III-a-e** via replacement of 4-nitrobenzyl chloroformate by phenyl isocyanate.
¹H NMR (CDC1₃, 300MHz) δ 7.58-7.26 (m, 8H), 7.08-6.98 (m, 2H), 6.60-6.52 (d, 1H), 6.00-5.86 (m, 1H), 5.48-5.32 (m, 2H), 4.44-4.32 (m, 1H), 3.91-3.50 (m, 5H), 3.11-2.02 (m, 9H), 1.96-1.49 (m, 12H);
ESI-MS *m*/*z* 531 (M⁺+1).

### Example 38

### Compound III-a-l

Compound **III-a-l** was prepared by following the procedure described for the synthesis of compound **III-a-e** via replacement of 4-nitrobenzyl chloroformate by 2-phenoxyacetyl chloride.
¹H NMR (CDCl₃, 300MHz) 7.52-7.26 (m, 7H), 7.00-6.89(m, 3H) 5.86-5.77 (m, 1H), 5.30-5.21 (m, 2H), 4.70-4.66 (d,2H), 4.58-4.40 (m, 1H),4.00-3.69 (m, 6H), 3.02-2.38 (m, 6H), 2.38-2.05 (m, 2H), 1.87-1.45 (m, 12H);
ESI-MS *m*/*z* 546.5 (M⁺+1).

The structural formulas of the compounds in the above Examples were listed in Table I.

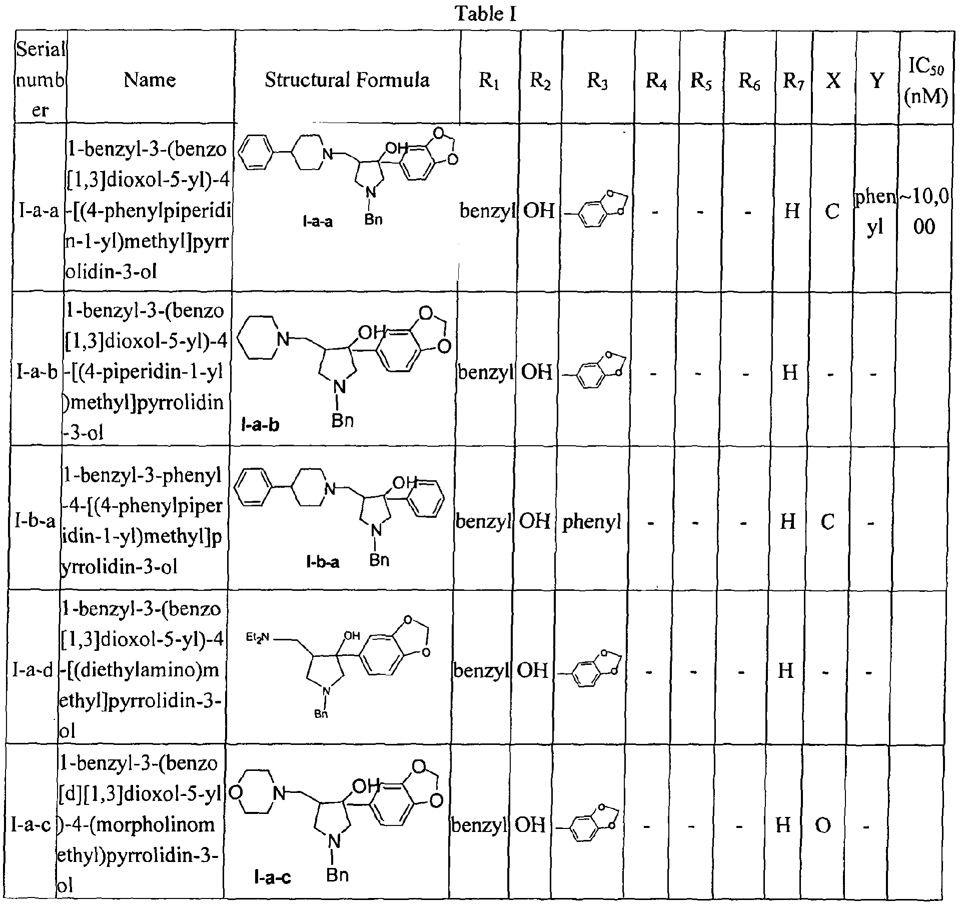

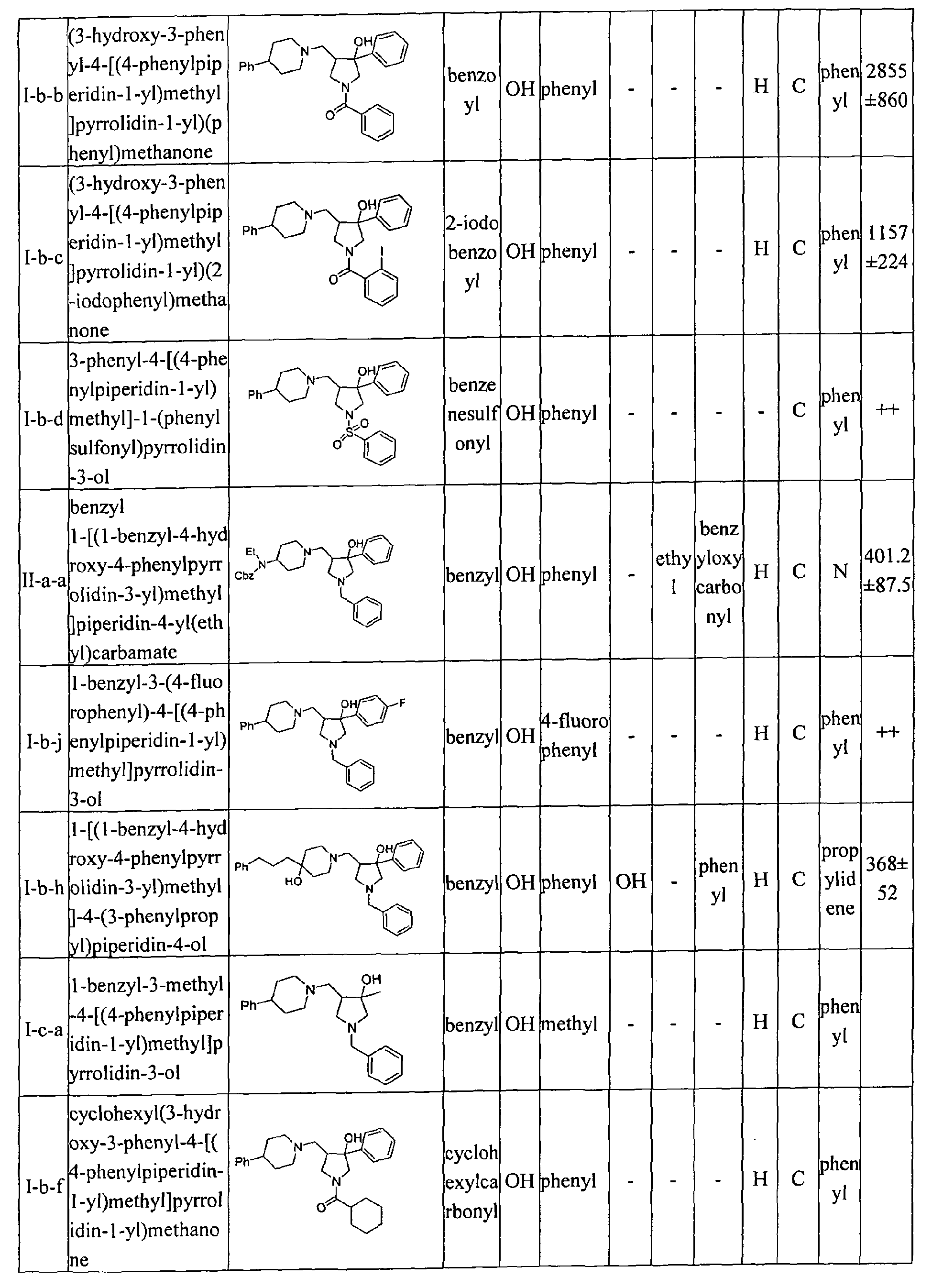

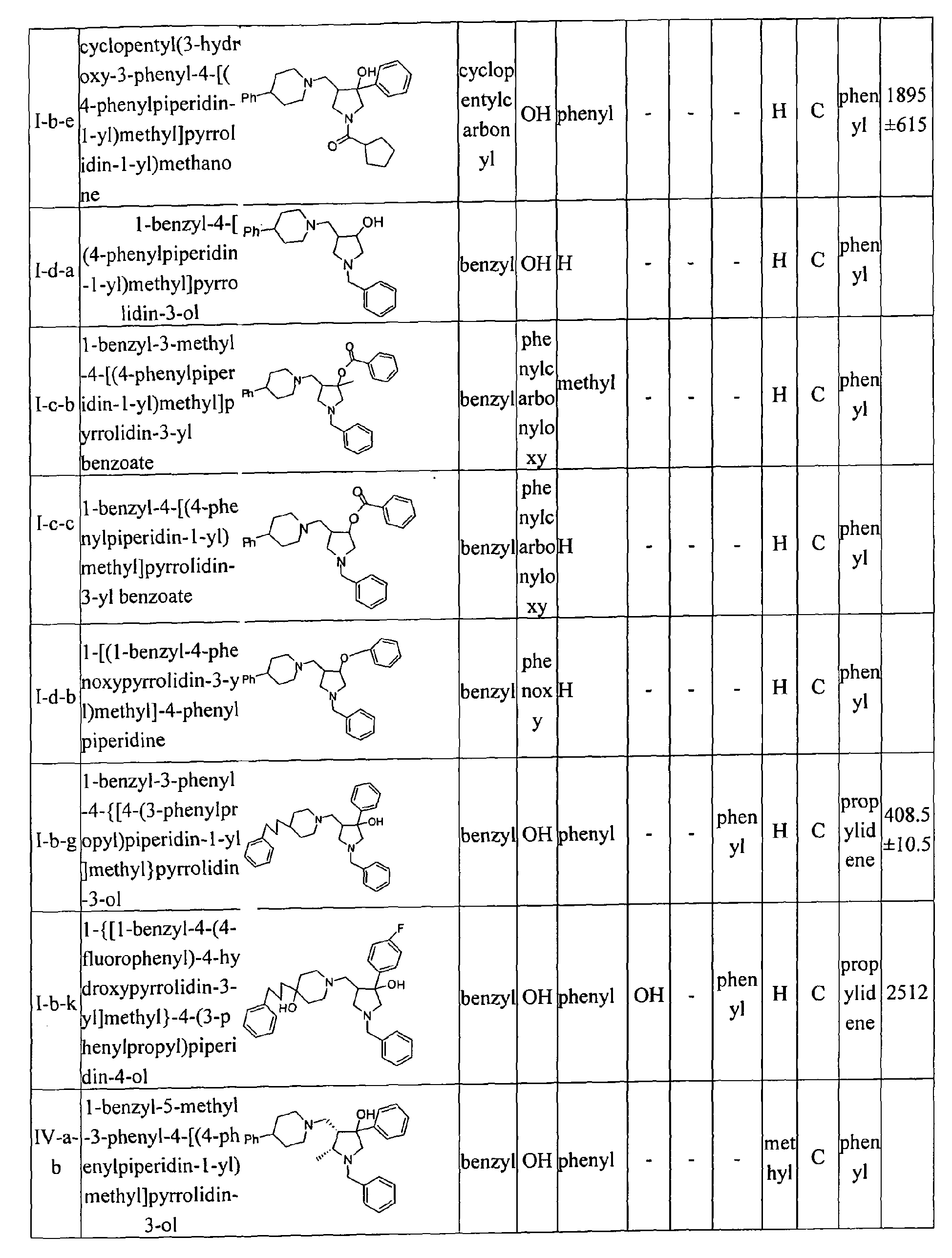

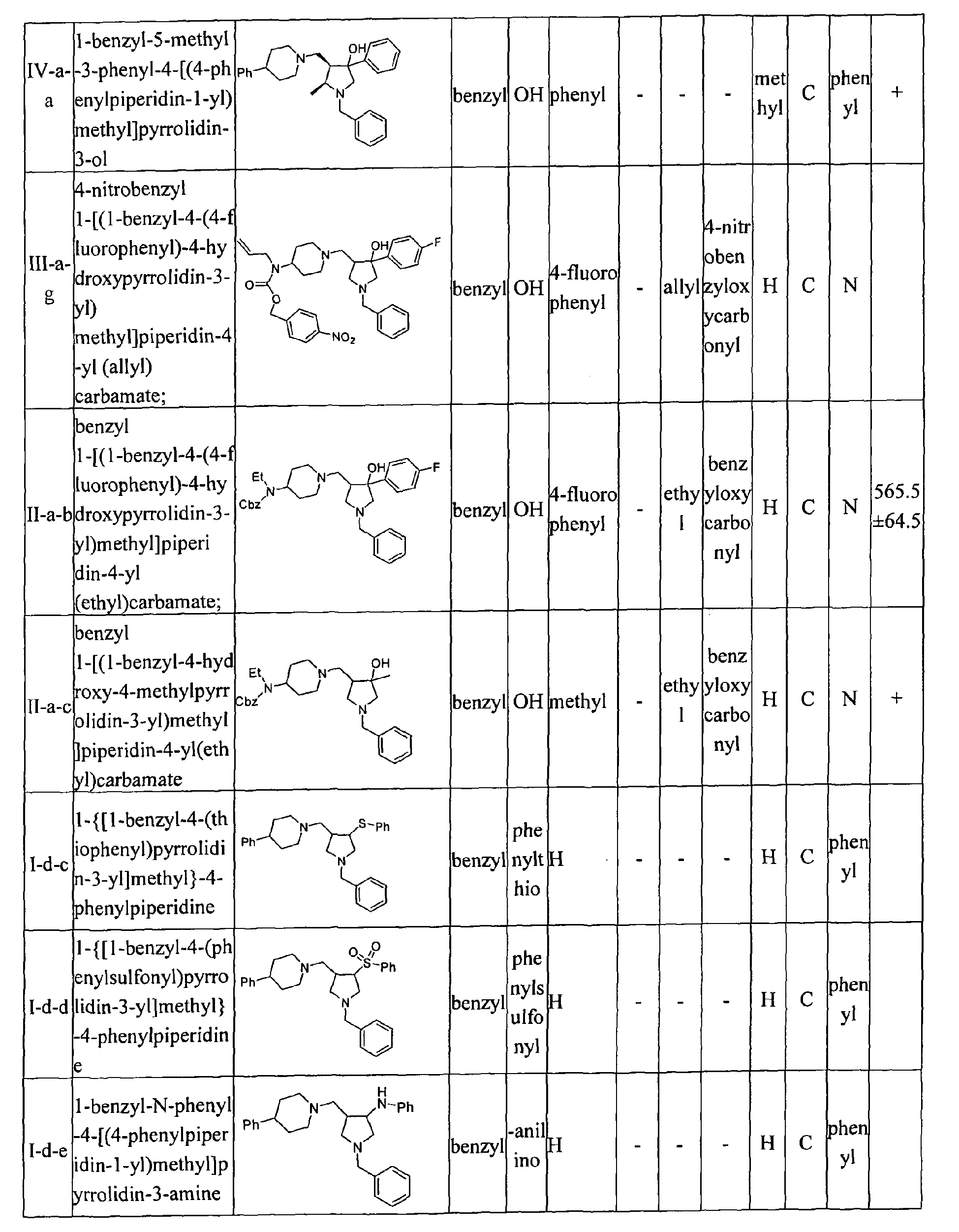

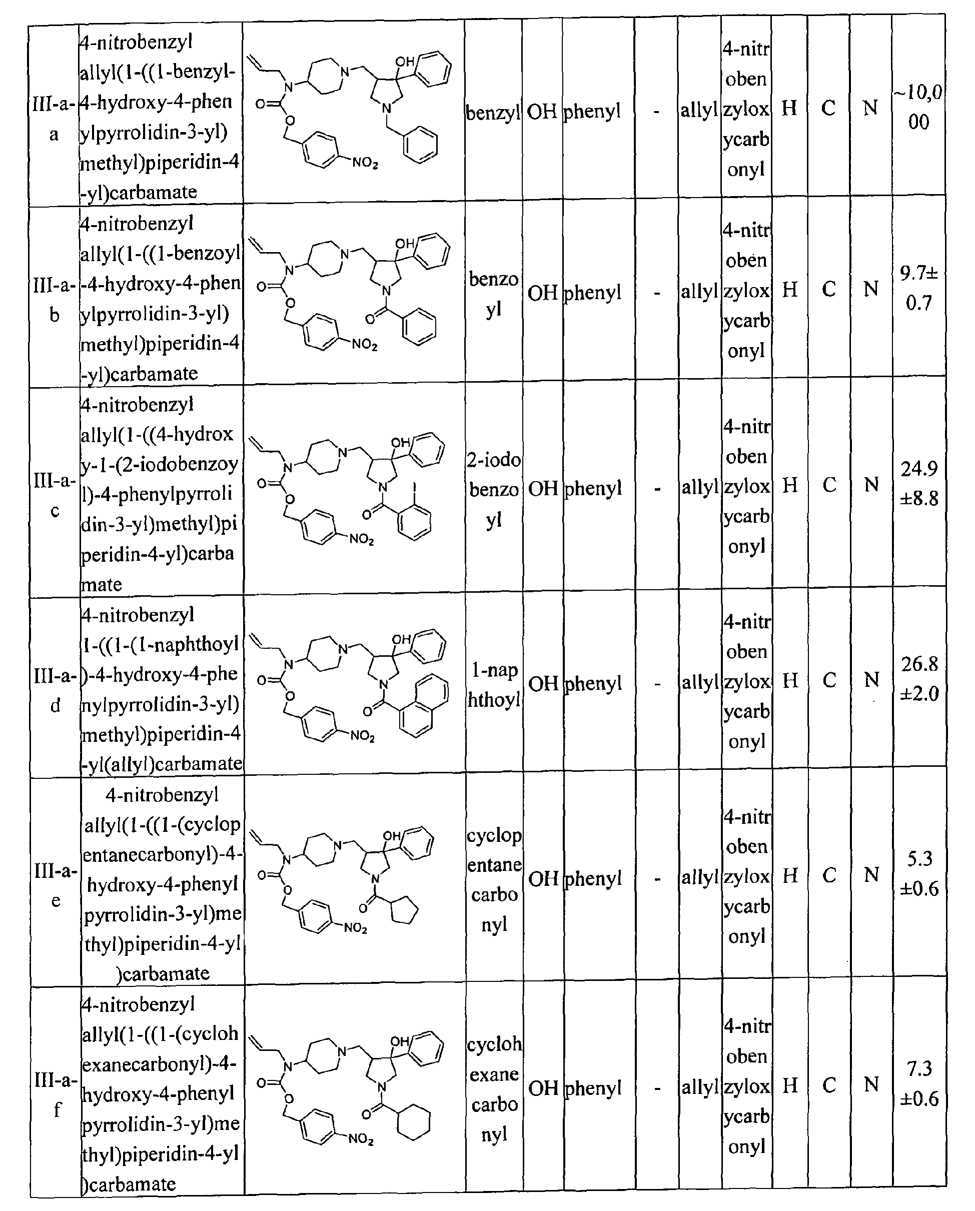

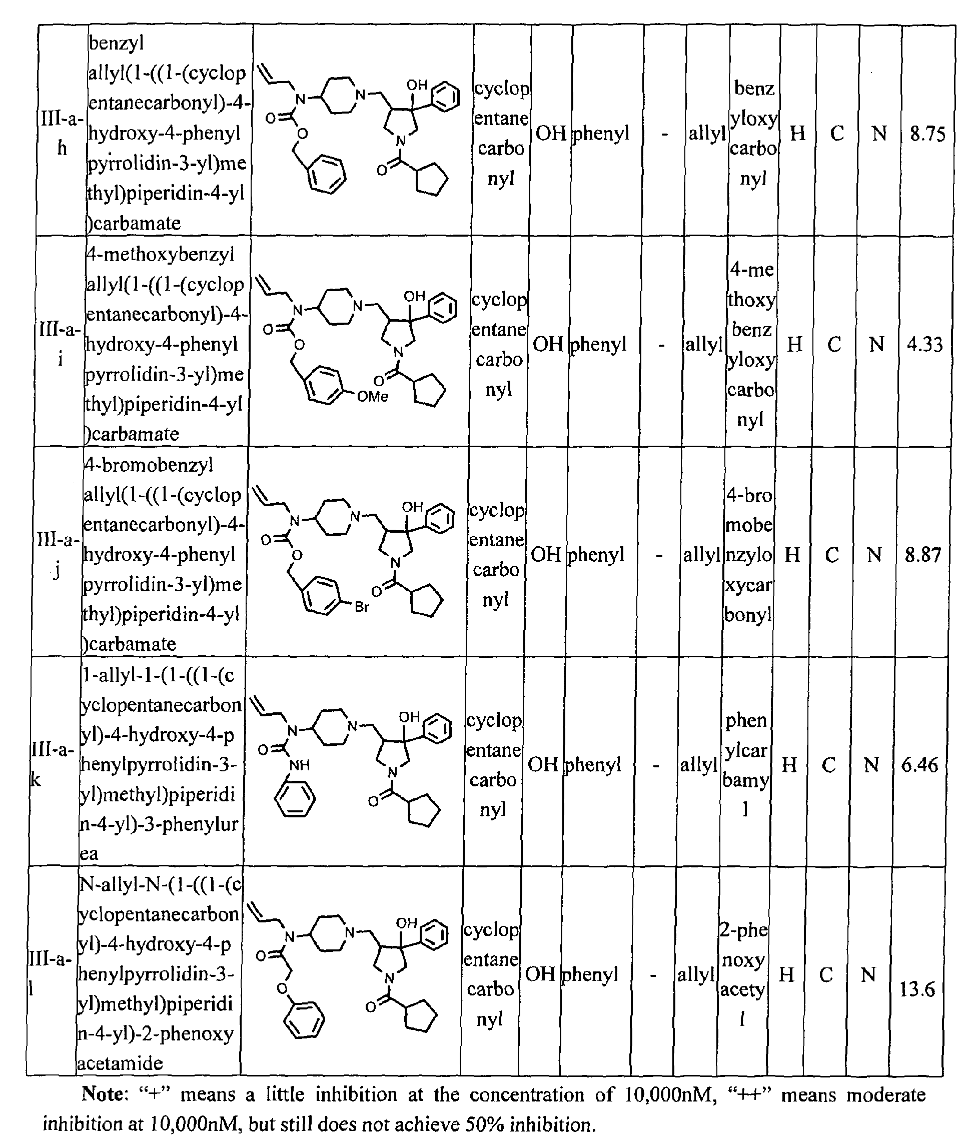

### Biological Testing

### [³⁵S]GTPγS binding assay

CCR5 receptor binds to agonists and changes its conformation which enables it to interact with and activate G protein, a heterotrimer which consists of subunits α and βγ. The capability of G protein α subunit binding to GTP depends on the effect between CCR5 and the agonists, therefore, the amount of α subunit bound GTP should reflect the agonists activity on the CCR5 receptor. In the GTPγS binding assay, [³⁵S]GTPγS is resistant to the GTPase activity of α subunit, and thus cannot be hydrolyzed when bound to G protein, making it accurate to reflect the receptor activation. Radiolabled [³⁵S]GTPγS can also serve as marker for detection in place of GTP. When CCR5 is not activated, α subunit is bound to GDP, but when CCR5 is activated, α subunit binds to [³⁵S]GTPγS, therefore, measurement of the number of the α subunit-bound ³⁵S-GTPγS can reflect the CCR5 activation level. When antagonists of the present invention are added to the system, the activation of the CCR5 receptor by agonists should be lowered.

### CCR5 activation of G-protein was measured according to assays below:

Permanent cell line expressing CHO of CCR5 (available at Euroscreen S.A., Belgium) was lysed by lysis buffer (5mM Tris HCl, pH 7.5, 5mM EDTA and 5mM EGTA), and centrifuged at 15,000 xg for 10 min. Cell membrane was resuspended with reaction buffer (5mM Tris HCl, pH 7.5, 5mM MgCl₂, 1mM EGTA, 100mM NaCl), and protein concentrations were determined using Bioford assay (Bio-rad). [³⁵S]GTPγS binding assay was performed in the 100 µl reaction buffer system, which contains 10ug membrane protein, 40uM GDP and 0.5 nM [³⁵S]GTPγS (1200 Ci / mmol). After adding the study compounds, the system was vortexed and placed on ice for 5 min, and then CCR5 agonist was added (10nM RANTES or 30 nM MIP1β). After vortexing, the reaction tubes were incubated at 30°C for 1h. After the reaction was complete, the reaction tubes were placed on ice and diluted with PBS to terminate the reaction. After suction through the GF/C filter membrane under vacuum, the membrane bound radioactivity was measured with a scintillation counter. Basal binding was measured without presence of agonist, and non-specific binding was measured in the presence of 10 mM nonisotopical GTPγS. The binding ratio of [³⁵S]GTPγS was calculated according to 100x[c.p.m.ₛₐₘₚₗₑ - c.p.m. non-specific]/ [c.p.m._{basal} - c.p.m. non-specific]. IC₅₀ was the compound concentration at which half of the [³⁵S]GTPγS binding induced by 10 nM RANTES or 30nM MIP1β was inhibited, and the value was obtained from the concentration-inhibition curve (6-7 points for concentrations of each compound).

### B. Chemotaxis assay

Cells expressing chemokine receptors can migrate towards the place where agonists are present when contacted with agonists, and thus the measurement of cell migration can reflect the interaction between receptors and agonists.. The procedures were as follows:
The assay is performed with a 48 well plate (AP48, Neuroprobe Inc., USA) and 8uM filter membrane (25 x 80mm). The filter membrane was pre-immersed in rat tail collagen for at least 2 h. The filter membrane was taken out and dried on a super-clean bench, then the filter membrane was immersed in 0.1% BSA-MEM. HEK 293 cells expressing human CCR5 receptors (293CCR5) (available at Euroscreen S.A., Belgium) were digested with D.T. for 1 min, and pelleted by centrifugation at 200 g. Resuspend the cells in 0.1% BSA-MEM, count and dilute the cell suspension to 3 x 10⁶ cells/ml. Fill each well with 26.5 µl of a chemokine dilution or 1% BSA MEM, place the filter membrane and cover a lid on the well, add 50 µl of the cells suspension to each well. When testing the compound antagonism, the compounds to be tested were added to the cell suspension, and pre-incubated at 37 °C for 20 min. Then place the plate in 37°C incubator, and incubate for 6h. Take out of the filter membrane and remove the cells in the well, fix the filter membrane in 4% polyformaldehyde solution at 4 °C overnight. The filter membrane was taken out the next day and stained with crystal violet for at least 2h, then wash with water and allow it to dry. Scan the filter membrane, and calculate the shades with Scion Image. Calculate and plot based on that the chemotactic number of the cells without antagonists is 100%.

### C. MTT cytotoxicity assay

Cells were prepared as single cell suspension, and cell densities were adjusted according to the cell size and cell features. Cells were inoculated with 100ul culture media into 96 well plates, and incubated in 37 °C incubator (5% CO₂, saturated humidity). The seeding densities were as follows: peripheral blood monouclear cells (PBMC) 10⁵ cells /well, Jurket 4x10⁴ cells / well. 24h after cell inoculation, the compounds to be tested were added and incubated with cells. 48h after inoculation, 10µl MTT (Sigma, USA, 5mg/ml, diluted with PBS and stored at -20 °C) was added to each well, and incubated for another 4h in 37 °C incubator. Then add 50 µl formazane solvent (10%SDS-5%isopropanol -0.01M HCl) and incubate overnight. Determine OD₅₇₀/OD₆₃₀ nm at spectrophotometer, and calculate CC₅₀ according to the inhibition curve.

### Results of biological testing

A. [³⁵S]GTPγS binding assay showed that the compounds of this invention are CCR5 antagonists, inhibiting the [³⁵S]GTPγS binding initiated by 10nM RANTES. The IC₅₀s are listed in the following table:

| Compounds | IC₅₀(nM)±S.E. |
|---|---|
| I-a-a | ~10,000 |
| I-b-b | 2855±60 |
| I-b-c | 1157±224 |
| I-b-d | ++ |
| I-b-e | 1895±615 |
| I-b-g | 408.5±10.5 |
| I-b-h | 368±52 |
| I-b-j | ++ |
| I-b-k | 2512 |
| II-a-a | 401.2±87.5 |
| II-a-b | 565.5±64.5 |
| II-a-c | + |
| III-a-a | ~10,000 |
| III-a-b | 9.7±0.7 |
| III-a-c | 24.9±8.8 |
| III-a-d | 26.8±2.0 |
| III-a-e | 5.3±0.6 |
| III-a-f | 7.3±0.6 |
| IV-a-a | + |
| III-a-h | 8.75 |
| 111-a-i | 4.33 |
| III-a-j | 8.87 |
| III-a-k | 6.46 |
| III-a-l | 13.6 |

| | |
|---|---|
| **Note:** "+" means a little inhibition at the concentration of 10,000nM, "++" means moderate inhibition at 10,000nM, but still does not achieve 50% inhibition. The lower the IC₅₀ is, the stronger the inhibition is. | |

We tested the effects of some compounds on the activation of CXCR4 and CCR1 receptors, and found that they (II-a-a, III-a-a, III-a-b, III-a-c, III-a-d, III-a-e, III-a-f) do not activate or antagonize these two receptors at the concentration of 10,000nM, therefore, they are specific CCR5 antagonists.

Moreover, mesylate of II-a-a has elevated water solubility (about 10 fold increase), and the activity and specificity remains the same as II-a-a, whose IC₅₀ is 341.5±72.5 nM.

For some compounds (including II-a-a and its mesylate), we tested their IC₅₀ of GTPγS binding initiated by 30 nM MIP-1β (another CCR5 agonist), and the GTPγS binding induced by MIP-1β is similar to that induced by inhibiting RANTES.
B. Chemotaxis assay demonstrated that the compounds of the present invention can inhibit cell chemotaxis induced by RANTES at low concentrations. The IC₅₀ of compound III-a-e on cell chemotaxis caused by 10 nM RANTES is about 30 nM.
C. Cytotoxicity study showed that tested compounds have no or low cytotoxicity. Compounds such as II-a-a, III-a-b, III-a-c, III-a-d, III-a-e, III-a-f, have no significant cytotoxicity at 10,000nM on cells, and the CC₅₀ is about 30,000 nM.

In general, the compounds described in this invention are potent CCR5 antagonists with high affinity. Since the cytotoxicity is low, the therapeutic index CC₅₀ /IC₅₀ is over 1000, therefore, it can be applied in clinical to treat diseases associated with CCR5, such as AIDS, autoimmune diseases and inflammatory diseases.

All the documents cited herein are incorporated into the invention as reference, as if each of them is individually incorporated. Further, it would be appreciated that, in the above teaching of invention, the skilled in the art could make certain changes or modifications to the invention, and these equivalents would still be within the scope of the invention defined by the appended claims of the application.

## Claims

1. A compound of Formula (I) or a pharmaceutically acceptable salt thereof: 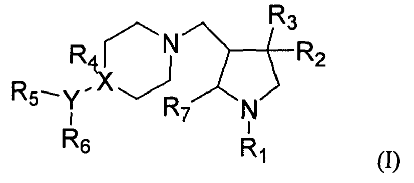 wherein:
R₁ is benzyl, benzoyl, cyclohexanecarbonyl, cyclopentanecarbonyl, phenylsulfonyl or naphthylcarbonyl, the groups are optionally substituted with 1-3 substituents independently selected from the group consisting of halogen, C₁₋₄ alkyl and C₁₋₄ alkoxy;
R₂ is hydroxyl, phenylcarbonyloxy, phenoxy, thiophenyl, anilino or phenylsulfonyl, wherein the benzene rings of the groups are optionally substituted with 1-3 substituents independently selected from the group consisting of halogen and C₁₋₄ alkyl;
R₃ is hydrogen, C₁₋₄ alkyl, phenyl or 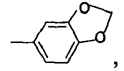 wherein the benzene rings of the groups are optionally substituted with 1-3 substituents independently selected from the group consisting of halogen and C₁₋₄ alkyl;
R₄ is hydrogen, hydroxyl or is absent;
R₇ is hydrogen, C₁-C₆ alkyl or phenyl;
X is oxygen or carbon or is absent;
provided that when X is oxygen or is absent, R₄, R₅, R₆ and Y are absent; or
provided that when X is carbon, Y is nitrogen, R₅ is C₁₋₆ alkyl or allyl and R₆ is selected from the group consisting of 4-nitro benzyloxycarbonyl, benzyloxycarbonyl, 4-halogen benzyloxycarbonyl, 4-methoxyl benzyloxycarbonyl, 4-methyl benzyloxycarbonyl, 4-trifluoromethyl benzyloxycarbonyl, 4-amino benzyloxycarbonyl; benzo[d][1,3]dioxol-5-yl methyloxycarbonyl, phenylsulfonyl, 4-methyl phenylsulfonyl, 2-phenoxyacetyl and phenylcarbamyl, or R₅, R₆ and Y together form phenyl or -R₈-phenyl, wherein R₈ is C₁₋₄ alkylidene.

2. The compound according to claim 1, wherein R₁ is benzyl, benzoyl, o-halo benzoyl, cyclohexanecarbonyl, cyclopentanecarbonyl, phenylsulfonyl or naphthylcarbonyl.

3. The compound according to claim 1, wherein R₂ is hydroxyl, phenylcarbonyloxy, phenoxy, thiophenyl, anilino or phenylsulfonyl.

4. The compound according to claim 1, wherein R₃ is hydrogen, C₁₋₄ alkyl, phenyl, 4-halogen phenyl, or

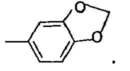

5. The compound according to claim 1, wherein X is oxygen or is absent, and R₄, R₅, R₆ and Y are absent.

6. The compound according to claim 1, wherein X is carbon, Y is nitrogen, R₅ is C₁-C₆ alkyl or allyl and R₆ is selected from the group consisting of 4-nitro benzyloxycarbonyl, benzyloxycarbonyl, 4-halogen benzyloxycarbonyl, 4-methoxyl benzyloxycarbonyl, 4-methyl benzyloxycarbonyl, 4-trifluoromethyl benzyloxycarbonyl, 4-amino benzyloxycarbonyl; benzo[d][1,3]dioxol-5-yl methyloxycarbonyl, phenylsulfonyl, 4-methyl phenylsulfonyl, 2-phenoxyacetyl and phenylcarbamyl; or R₅, R₆ and Y together form phenyl or -CH₂CH₂CH₂-phenyl.

7. The compound according to claim 1, wherein R₇ is hydrogen, C₁-C₃ alkyl or phenyl.

8. The compound according to claim 1, wherein the compound is represented by the structural formula III, 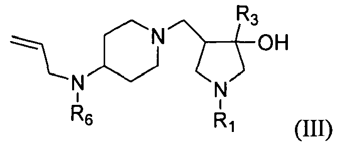 wherein,
R₁ is benzyl, benzoyl, cyclohexanecarbonyl, cyclopentanecarbonyl, phenylsulfonyl or naphthylcarbonyl, the groups are optionally substituted with 1-3 substituents independently selected from the group consisting of halogen, C₁₋₄ alkyl and C₁₋₄ alkoxy;
R₃ is hydrogen, C₁₋₄ alkyl, phenyl or 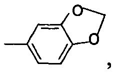 wherein the benzene rings of the groups are optionally substituted with 1, 2 or 3 substituents independently selected from the group consisting of halogen and C₁₋₄ alkyl;
R₆ is selected from the group consisting of 4-nitro benzyloxycarbonyl, benzyloxycarbonyl, 4-halogen benzyloxycarbonyl, 4-methoxyl benzyloxycarbonyl, 4-methyl benzyloxycarbonyl, 4-trifluoromethyl benzyloxycarbonyl, 4-amino benzyloxycarbonyl; benzo[d][1,3]dioxol-5-yl methyloxycarbonyl, phenylsulfonyl, 4-methyl phenylsulfonyl, 2-phenoxyacetyl and phenylcarbamyl.

9. The compound according to claim 1, which is independently selected from:
1-benzyl-3-(benzo[1,3]dioxol-5-yl)-4-[(4-phenylpiperidin-1-yl)methyl]pyrrolidin-3-ol;
1-benzyl-3-(benzo[1,3]dioxol-5-yl)-4-[(4-piperidin-1-yl)methyl]pyrrolidin-3-ol;
1-benzyl-3-phenyl-4-[(4-phenylpiperidin-1-yl)methyl]pyrrolidin-3-ol;
1-benzyl-3-(benzo [1,3]dioxol-5-yl)-4-[(diethylamino)methyl]pyrrolidin-3-ol;
1-benzyl-3-(benzo[d][1,3]dioxol-5-yl)-4-(morpholinomethyl)pyrrolidin-3-ol;
(3-hydroxy-3-phenyl-4-[(4-phenylpiperidin-1-yl)methyl]pyrrolidin-1-yl)(phenyl)methanone;
(3-hydroxy-3-phenyl-4-[(4-phenylpiperidin-1-yl)methyl]pyrrolidin-1-yl)(2-iodophenyl)methanon e;
3-phenyl-4-[(4-phenylpiperidin-1-yl)methyl]-1-(phenylsulfonyl)pyrrolidin-3-ol; benzyl 1-[(1-benzyl-4-hydroxy-4-phenylpyrrolidin-3-yl)methyl]piperidin-4-yl (ethyl)carbamate;
1-benzyl-3-(4-fluorophenyl)-4-[(4-phenylpiperidin-1-yl)methyl]pyrrolidin-3-ol;
1-[(1-benzyl-4-hydroxy-4-phenylpyrrolidin-3-yl)methyl]-4-(3-phenylpropyl)piperidin-4-ol;
1-benzyl-3-methyl-4-[(4-phenylpiperidin-1-yl)methyl]pyrrolidin-3-ol;
cyclohexyl(3-hydroxy-3-phenyl-4-[(4-phenylpiperidin-1-yl)methyl]pyrrolidin-1-yl)methanone;
cyclopentyl(3-hydroxy-3-phenyl-4-[(4-phenylpiperidin-1-yl)methyl]pyrrolidin-1-yl)methanone;
1-benzyl-4-[(4-phenylpiperidin-1-yl)methyl]pyrrolidin-3-ol;
1-benzyl-3-methyl-4-[(4-phenylpiperidin-1-yl)methyl]pyrrolidin-3-yl benzoate;
1-benzyl-4-[(4-phenylpiperidin-1-yl)methyl]pyrrolidin-3-yl benzoate;
1-[(1-benzyl-4-phenoxypyrrolidin-3-yl)methyl]-4-phenylpiperidine;
1-benzyl-3-phenyl-4-{[4-(3-phenylpropyl)piperidin-1-yl]methyl}pyrrolidin-3-ol;
1-{[1-benzyl-4-(4-fluorophenyl)-4-hydroxypyrrolidin-3-yl]methyl}-4-(3-phenylpropyl)piperidin-4-ol;
1 -benzyl-5-methyl-3-phenyl-4-[(4-phenylpiperidin- I -yl)methyl]pyrrolidin-3-ol;
4-nitrobenzyl 1-[(1-benzyl-4-(4-fluorophenyl)-4-hydroxypyrrolidin-3-yl) methyl]piperidin-4-yl (allyl) carbamate;
benzyl 1-[(1-benzyl-4-(4-fluorophenyl)-4-hydroxypyrrolidin-3-yl)methyl]piperi din-4-yl (ethyl)carbamate;
benzyl 1-[(1-benzyl-4-hydroxy-4-methylpyrrolidin-3-yl)methyl]piperidin-4-yl (ethyl)carbamate;
1-{[1-benzyl-4-(thiophenyl)pyrrolidin-3-yl]methyl}-4-phenylpiperidine;
1-{[1-benzyl-4-(phenylsulfonyl)pyrrolidin-3-yl]methyl}-4-phenylpiperidine;
1-benzyl-N-phenyl-4-[(4-phenylpiperidin-1-yl)methyl]pyrrolidin-3-amine;
4-nitrobenzyl allyl(1-((1-benzyl-4-hydroxy-4-phenylpyrrolidin-3-yl)methyl)piper idin-4-yl)carbamate;
4-nitrobenzyl allyl(1-((1-benzoyl-4-hydroxy-4-phenylpyrrolidin-3-yl)methyl)pip eridin-4-yl)carbamate;
4-nitrobenzyl allyl(1-((4-hydroxy-1-(2-iodobenzoyl)-4-phenylpyrrolidin-3-yl)me thyl)piperidin-4-yl)carbamate;
4-nitrobenzyl allyl(1-((1-(2-naphthoyl)-4-hydroxy-4-phenylpyrrolidin-3-yl)meth yl)piperidin-4-yl)carbamate;
4-nitrobenzyl allyl(1-((1-(cyclopentanecarbonyl)-4-hydroxy-4-phenylpyrrolidin -3-yl)methyl)piperidin-4-yl)carbamate;
4-nitrobenzyl allyl(I-((I-(cyclohexanecarbonyl)-4-hydroxy-4-phenylpyrrolidin -3-yl)methyl)piperidin-4-yl)carbamate;
benzyl allyl(1-((1-(cyclopentanecarbonyl)-4-hydroxy-4-phenylpyrrolidin-3-yl)me thyl)piperidin-4-yl)carbamate;
4-methoxybenzyl allyl(1-((1-(cyclopentanecarbonyl)-4-hydroxy-4-phenylpyrrol idin-3-yl)methyl)piperidin-4-yl)carbamate;
4-bromobenzyl allyl(1-((1-(cyclopentanecarbonyl)-4-hydroxy-4-phenylpyrrolid in-3-yl)methyl)piperidin-4-yl)carbamate;
1-allyl-1-(1-((1-(cyclopentanecarbonyl)-4-hydroxy-4-phenylpyrrolidin-3-yl)methyl)piperidin-4-y 1)-3-phenylurea;
N-allyl-N-(1-((1-(cyclopentanecarbonyl)-4-hydroxy-4-phenylpyrrolidin-3-yl)methyl)piperidin-4-yl)-2-phenoxyacetamide.

10. A pharmaceutical composition comprising the compound of claim 1 in combination with a pharmaceutically acceptable carrier.

11. The use of the compound according to claim 1 for the preparation of a medicament.

12. A compound of formula II, 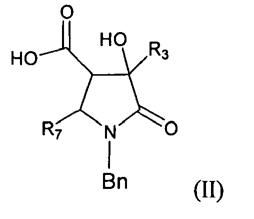 wherein,
R₃ is hydrogen, C₁₋₄ alkyl, phenyl or 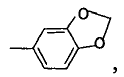 wherein the benzene rings of the groups are optionally substituted with 1, 2 or 3 substituents independently selected from the group consisting of halogen and C₁₋₄ alkyl;
R₇ is hydrogen, C₁₋₆ alkyl or phenyl.
